# EUROPEAN PATENT SPECIFICATION

(11) **EP 3 158 086 B1**
(45) Date of publication and mention of the grant of the patent: **24.03.2021**
(21) Application number: 15809484.7
(22) Date of filing: 19.06.2015
(51) Int. Cl.: C12Q 1/6886, A61K 31/5377, G01N 33/50

(54) **BIOMARKERS FOR RESPONSE TO EZH2 INHIBITORS**
BIOMARKER FÜR REAKTION AUF EZH2-HEMMER
BIOMARQUEURS DE LA REPONSE AUX INHIBITEURS D'EZH2

(30) Priority: 19.06.2014 US 201462014594 P
(43) Date of publication of application: 26.04.2017
(73) Proprietor: Memorial Sloan-Kettering Cancer Center, New York, NY 10065 (US)
(72) Inventor: LEVINE, Ross, New York, NY 10065 (US); LAFAVE, Lindsay, New York, NY 10021 (US); ABDEL-WAHAB, Omar, NewYork, NY 10065 (US)
(74) Representative: Barker Brettell LLP
(86) International application number: PCT/US2015/036677
(87) International publication number: WO 2015/196064

(56) References cited:
- WO-A1-99/25373
- WO-A1-2012/144220
- WO-A2-2013/049770
- US-A1- 2014 011 696
- HUI SHEN ET AL: "Interplay between the Cancer Genome and Epigenome", CELL, vol. 153, no. 1, 1 March 2013 (2013-03-01) , pages 38-55, XP055316032, US ISSN: 0092-8674, DOI: 10.1016/j.cell.2013.03.008
- MARIE SCHOUMACHER ET AL: "Uveal melanoma cells are resistant to EZH2 inhibition regardless of BAP1 status", NATURE MEDICINE, vol. 22, no. 6, 1 June 2016 (2016-06-01), pages 577-578, XP055430521,
- LINDSAY LAFAVE ET AL: "Reply to "Uveal melanoma cells are resistant to EZH2 inhibition regardless of BAP1 status"", NATURE MEDICINE, vol. 22, no. 6, 1 June 2016 (2016-06-01), pages 578-579, XP055430526,
- O ABDEL-WAHAB ET AL: "The ASXL-BAP1 axis: new factors in myelopoiesis, cancer and epigenetics", LEUKEMIA., vol. 27, no. 1, 9 October 2012 (2012-10-09), pages 10-15, XP055430251, US ISSN: 0887-6924, DOI: 10.1038/leu.2012.288
- KEMP, CD ET AL.: 'Polycomb Repressor Complex-2 Is A Novel Target For Mesothelioma Therapy' CLIN CANCER RES. vol. 18, no. 1, 01 January 2012, ISSN 1078-0432 pages 77 - 90, XP055247285
- CHEN, X ET AL.: 'MicroRNA-124a Is Epigenetically Regulated And Acts As A Tumor Suppressor By Controlling Multiple Targets In Uveal Melanoma' INVEST OPHTHALMOL VIS SCI vol. 54, no. 3, 01 March 2013, ISSN 1552-5783 pages 2248 - 2256, XP055247287
- SHEN, Y ET AL.: 'Expression And Significance Of Histone H3K27 Demethylases In Renal Cell Carcinoma.' BMC CANCER vol. 12, no. 470, 12 October 2012, ISSN 1471-2407 page 470, XP021128441
- BACHMANN, IM ET AL.: 'Ezh2 Expression Is Associated With High Proliferation Rate And Aggressive Tumor Subgroups In Cutaneous Melanoma And Cancers Of The Endometrium, Prostate, And Breast.' J CLIN ONCOL. vol. 24, no. 2, 10 January 2006, ISSN 0732-183X pages 268 - 273, XP055247289
- BATTAGLIA, A. ET AL.: 'The Importance Of Multidisciplinary Approach In Early Detection Of BAP1 Tumor Predisposition Syndrome: Clinical Management And Risk Assessment.' CLIN MED INSIGHTS ONCOL vol. 8, 28 April 2014, pages 37 - 47, XP055247292

## Description

### CROSS-REFERENCE TO RELATED APPLICATIONS

This application claims priority to U.S. Provisional Patent Application Serial No. 62/014,594, filed June 19, 2014.

### 1. INTRODUCTION

This present invention relates to biomarkers that may be used to evaluate the likelihood that an EZH2 inhibitor would produce an anti-cancer effect in a subject. As such, these biomarkers may be used in methods of treating cancer patients.

### 2. BACKGROUND OF THE INVENTION

BRCA1 associated protein-1 (BAP1) is an ubiquitin carboxy-terminal hydrolase that is involved in the removal of ubiquitin from proteins. BAP1 binds to the breast cancer type 1 susceptibility protein (BRCA1) via the RING finger domain of BRCA1 and can act as a tumor suppressor. BAP1 is involved in the regulation of transcription, regulation of cell cycle and growth, response to DNA damage and chromatin dynamics. Genome-sequencing studies have shown that germline mutations in *BAP1* can be associated with tumor predisposition syndrome (TPDS), which involves increased risk of cancers including malignant mesothelioma, uveal melanoma and cutaneous melanoma. Further studies have identified germline BAP1 mutations associated with other cancers including lung adenocarcinoma and renal cell carcinoma. The prognosis of some patients with BAP1-mutations is quite poor with no identified effective treatments, as many patients with malignant mesothelioma will die from their disease. BAP1 mutations in patients with renal cell carcinoma predict for poor prognosis and BAP1 mutations in uveal melanoma patients predict for a higher risk group and metastasis.

Enhancer of zeste homolog 2 (EZH2), is a member of the Polycomb-group (PcG) family, members of which are involved in the regulation of the transcriptional state of genes by methylation of histone proteins. Drugs have been developed that specifically target EZH2 and the effect of such drugs in patients with a variety of tumors has been an active area of investigation. EZH2 inhibitors are currently being tested clinically in lymphoma patients with EZH2-activating mutations. Accordingly, there is a need in the art for treatments for patients with BAP1-mutations and biomarkers that will be useful to determine when an EZH2 inhibitor should be used to treat a cancer.

For reference only, US 2014/011696 describes a kit with BAP1 directed primers or antibodies. WO 2012/144220 describes an increased expression of EZH2 as a diagnostic marker in some cancers. WO 2013/049770 describes specific mutations of EZH2 as a marker for the response to treatment with EZH2 inhibitors. Hui Shen et al. (2013) discusses the interplay between the cancer genome and the epigenome and makes reference to BAP1.

### 3. SUMMARY OF THE INVENTION

In a first aspect of the present invention, there is provided an EZH2 inhibitor for use in the treatment of a cancer in a subject in need thereof, wherein the expression of a BAP1 biomarker in the cancer of the subject is absent or expressed at a lower level than a BAP1 reference control level, wherein the BAP1 reference control level is the expression level of the BAP1 biomarker in a reference control sample; wherein the cancer is selected from the group consisting of malignant mesotheliomas, uveal melanomas, renal cell carcinoma, cutaneous melanomas, lung cancer, breast cancer, ovarian cancer, non-melanoma skin cancer, meningioma, cholangiocarcinoma, leiomyosarcoma, neuroendocrine tumors, pancreatic cancer, paraganglioma, malignant fibrous histiocytoma, melanocytic BAP1-mutated atypical intradermal tumors (MBAITs), acute myeloid leukemia, myelodysplastic syndromes, and bladder cancer; and wherein the EZH2 inhibitor is selected from the group consisting of UNC1999, 3-deazaneplanocin A, EI1, EPZ-6438, EPZ-5676, GSK343, EPZ005687, EPZ011989, and GSK126.

In another aspect of the present invention, there is provided an EZH2 inhibitor for use in a method for treating a cancer in a subject in need thereof, comprising determining the expression of a BAP1 biomarker in one or more cells of the cancer of the subject, where if the BAP1 biomarker is absent or expressed at lower levels in the cancer, as compared to a BAP1 reference control level, then administering a therapeutically effective amount of the EZH2 inhibitor to the subject to produce an anti-cancer effect, wherein the BAP1 reference control level is the expression level of the BAP1 biomarker in a reference control sample; wherein the cancer is selected from the group consisting of malignant mesotheliomas, uveal melanomas, renal cell carcinoma, cutaneous melanomas, lung cancer, breast cancer, ovarian cancer, non-melanoma skin cancer, meningioma, cholangiocarcinoma, leiomyosarcoma, neuroendocrine tumors, pancreatic cancer, paraganglioma, malignant fibrous histiocytoma, melanocytic BAP1-mutated atypical intradermal tumors (MBAITs), acute myeloid leukemia, myelodysplastic syndromes, and bladder cancer; and wherein the EZH2 inhibitor is selected from the group consisting of UNC1999, 3-deazaneplanocin A, EI1, EPZ-6438, EPZ-5676, GSK343, EPZ005687, EPZ011989, and GSK126.

In another aspect of the present invention, there is provided a method for determining whether an anti-cancer effect is likely to be produced in a cancer by an EZH2 inhibitor, comprising obtaining a sample of a cancer, and determining, in the sample, the expression level of an BAP1 biomarker, where if the BAP1 biomarker is absent or expressed at a lower level in the cancer, as compared to a BAP1 reference control level, it is more likely that the EZH2 inhibitor would have an anti-cancer effect on the cancer, wherein the BAP1 reference control level is the expression level of the BAP1 biomarker in a reference control sample; wherein the cancer is selected from the group consisting of malignant mesotheliomas, uveal melanomas, renal cell carcinoma, cutaneous melanomas, lung cancer, breast cancer, ovarian cancer, non-melanoma skin cancer, meningioma, cholangiocarcinoma, leiomyosarcoma, neuroendocrine tumors, pancreatic cancer, paraganglioma, malignant fibrous histiocytoma, melanocytic BAP1-mutated atypical intradermal tumors (MBAITs), acute myeloid leukemia, myelodysplastic syndromes, and bladder cancer; and wherein the EZH2 inhibitor is selected from the group consisting of UNC1999, 3-deazaneplanocin A, EI1, EPZ-6438, EPZ-5676, GSK343, EPZ005687, EPZ011989, and GSK126.

In another aspect of the invention, there is provided a method of predicting the sensitivity of a cancer to an EZH2 inhibitor, comprising obtaining a sample of the cancer and determining the expression level of a BAP1 protein biomarker in cells of the sample, wherein if the BAP1 biomarker is absent or reduced in expression level compared to a BAP1 reference control level, then the cancer is predicted to be sensitive to an EZH2 inhibitor, wherein the BAP1 reference control level is the expression level of the BAP1 biomarker in a reference control sample; wherein the cancer is selected from the group consisting of malignant mesotheliomas, uveal melanomas, renal cell carcinoma, cutaneous melanomas, lung cancer, breast cancer, ovarian cancer, non-melanoma skin cancer, meningioma, cholangiocarcinoma, leiomyosarcoma, neuroendocrine tumors, pancreatic cancer, paraganglioma, malignant fibrous histiocytoma, melanocytic BAP1-mutated atypical intradermal tumors (MBAITs), acute myeloid leukemia, myelodysplastic syndromes, and bladder cancer; and wherein the EZH2 inhibitor is selected from the group consisting of UNC1999, 3-deazaneplanocin A, EI1, EPZ-6438, EPZ-5676, GSK343, EPZ005687, EPZ011989, and GSK126.

In another aspect of the invention, there is provided use of a kit in the method for determining whether an anti-cancer effect is likely to be produced in a cancer by an EZH2 inhibitor as defined above, wherein the kit comprises a means for detecting a BAP1 biomarker. In certain embodiments, the means for detecting a BAP1 biomarker comprises one or more packaged primers, probes, arrays/microarrays, biomarker-specific antibodies and/or beads. In certain embodiments, the means for detecting a BAP1 biomarker comprises one or more antibodies, or antigen binding fragment thereof, for detecting a BAP1 biomarker. In certain embodiments, the kit further comprises one or more primers, probe, arrays/microarray, biomarker-specific antibody and/or bead for detecting EZH2 and/or SUZ12 expression.

### 4. BRIEF DESCRIPTION OF FIGURES

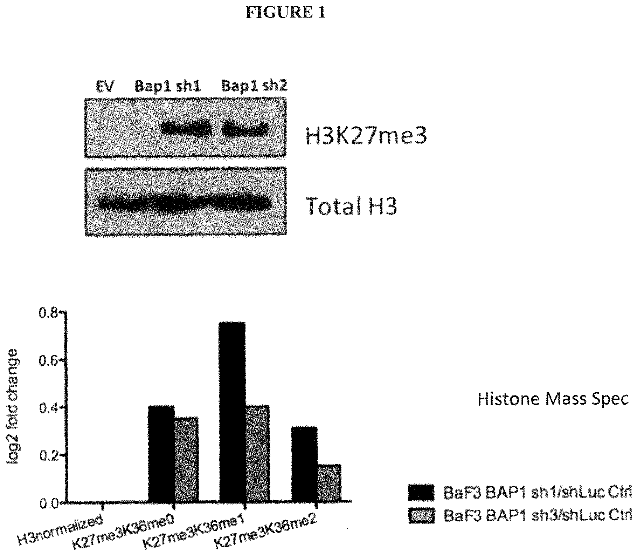

FIGURE 1. *BAP1* loss upregulated histone H3K27me3 *in vitro.*

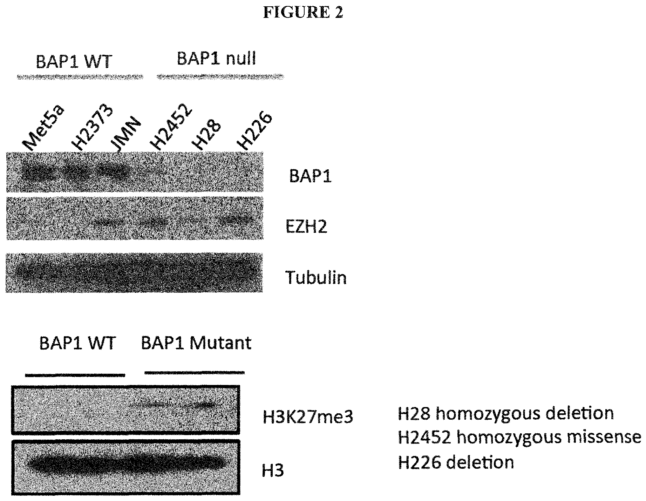

FIGURE 2. EZH2 was overexpressed in BAP1-mutant mesothelioma cells.

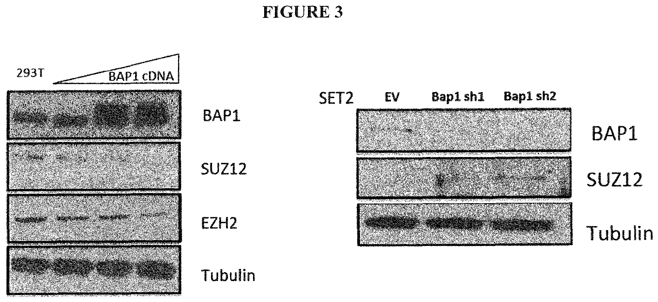

FIGURE 3. Gain/loss of *BAP1* expression resulted in altered PRC2 subunit expression.

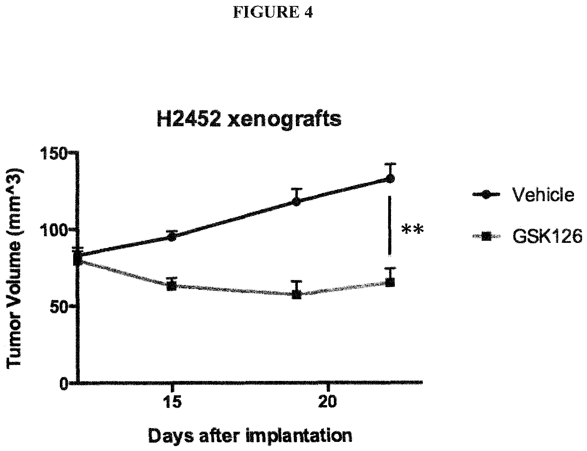

FIGURE 4. Inhibition of EZH2 decreased tumor volume in mesothelioma xenografts.

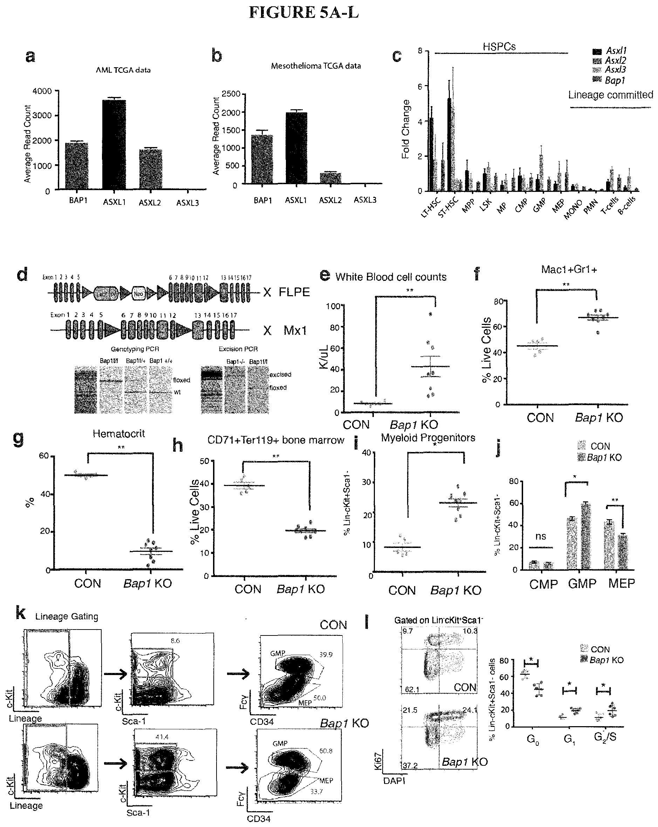

FIGURE 5A-L. Characterization of conditional hematopoietic deletion of *Bap1.* (a) Average gene expression of BAP1, ASXL1, ASXL2, and ASXL3 in TCGA AML (acute myeloid leukemia) and (b) mesothelioma patients as expressed as a mathematical mean with standard error of normalized read counts. (c) *Bap1* expression by qRT-PCR in purified populations of hematopoietic cells in C57/B6H mice. LT-HSC, long term hematopoietic stem cells (HSCs), (Lin⁻Sca-1⁺c-Kit⁺CD150⁺CD48⁻); ST-HSC, short term HSCs (Lin⁻Sca-1⁺c-Kit⁺CD150⁺CD48⁺); MPP, mulitpotent progenitor, (Lin⁻Sca-1⁺c-Kit⁺CD150⁻CD48⁺); LSK, Lin⁻Sca-1⁺c-Kit⁺; MP, myeloid progentiors (Lin⁻Sca-1⁻c-Kit⁺), GMP, Granulocyte Macrophage Progenitors (Lin⁻Sca-1⁻c-Kit⁺ CD34⁺Fcγ⁺); CMP, Common Myeloid Progenitors (Lin⁻ Sca-1⁻c-Kit⁺ CD34⁺Fcγ^{lo}); MEP; Macrophage Erythroid Progenitors (Lin⁻Sca-1⁻c-Kit⁺ CD34⁻PcΥ⁻), MONO; monocytes (Mac1⁺Gr1⁻), PMN; (polymorphonuclear neutrophil, Mac1⁺Gr1⁺), T cells, CD3⁺; and B cells, B220⁺. (d) *Bap1* targeting scheme in murine embryonic stem cells obtained from the EUCOMM consortium. After chimera generation, mice were crossed with transgenic FLPE mice to excise the premature stop cassette. Mice were then crossed to Mx1-*Cre* transgenic mice. Genotyping schemes confirming genotype and excision 4 weeks post-polyIpolyC (pIpC) treatment. (e) Enumeration of white blood cells in peripheral blood in control and *Bap1* KO mice after treatment with (pIpC) to induce excision and (f) flow cytometric enumeration of myeloid cells (Mac1⁺Gr1⁺). (g) Hematocrit percentages in peripheral blood and (h) flow cytometric enumeration of red blood cell precursors (CD71⁺Ter119⁺) in control and *Bap1* KO mouse bone marrow after pIpC-induced excision. (i) Relative frequencies of control and *Bap1* KO bone marrow myeloid progenitor populations (Lin⁻c-Kit⁺Sca1⁻). Cells were gated on live lineage-negative populations. (j) Relative quantification of bone marrow myeloid progenitor cell populations (GMP, CMP, MEP) in control and *Bap1* KO mice. (k) Flow plots from example control and bone marrow animals to demonstrate progenitor and GMP expansion. (1) Flow cytometric enumeration of cycling progenitor cells (Ki67/DAPI stain); for all experiments: n=5 CON mice and n=8 *Bap1* KO mice.

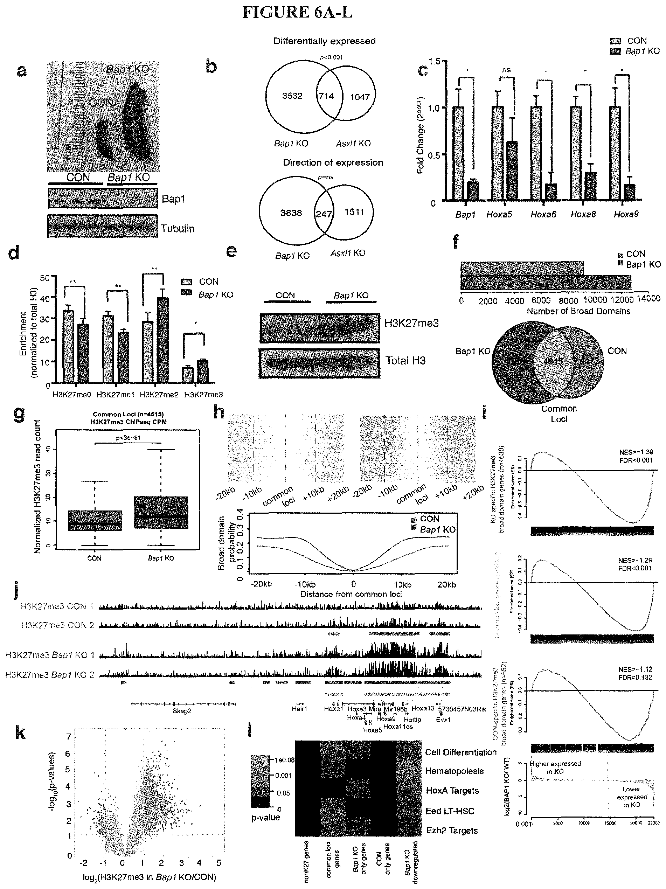

FIGURE 6A-L. *Bap1* deletion leads to differential pathway activation, as compared to *Asxl1* loss, and increased H3K27me3. (a) Spleen images three weeks after conditional deletion of *Bap1* and verification of *Bap1* deletion by Western blot of control (littermate *Bap1f*/*f* mice, CON) and *Bap1* knockout (*Mx1-Cre Bap1f*/*f* mice, *Bap1* KO) bone marrow. (b) Venn diagrams comparing myeloid progenitor gene expression in *Bap1* and *Asxl1* KO mice, p<0.05; comparisons include gene overlap and genes changing in the same direction. (c) Quantitative real time qPCR (qRT-PCR) of the *HoxA* cluster in sorted granulocyte-macrophage progenitors (GMPs; Lin⁻ c-Kit⁺Sca1⁻CD34⁺ Fcγ⁺); from *Bap1* KO and control mice (n=3). (d) Mass spectrometric analysis of purified histones from c-Kit⁺ enriched bone marrow cells from *Bap1* KO and controls normalized to total histone H3. (e) Western blot of H3K27me3 and total H3 in purified histones from *Bap1* KO and control bone marrow. (f) Number of H3K27me3 broad domains that are called in the CON and *Bap1 KO* samples. Venn diagram showing unique and overlapping broad domain. (g) Box plot showing normalized H3K27me3 reads in c-Kit enriched bone marrow cells (n=2) (h) Plotting broad domain density as a function of distance from an H3K27me3 domain that was called in both CON and *Bap1* KO samples. (i) GSEA demonstrating gene expression correlations to downregulated genes. (j) Local plot of H3K27me3 ChIP-seq at the HOXA locus. (k) Peak calls from H3K27me3 ChIP-Seq in sorted GMP cells displayed in a volcano plot as displayed by ratio (KO/CON) vs. p-value. (1) Significance of specified gene signatures to H3K27me3 bound genes and RNA-Seq. Statistics were calculated with Student t-test; *p<0.05, **p<0.005; ± SEM values reported.

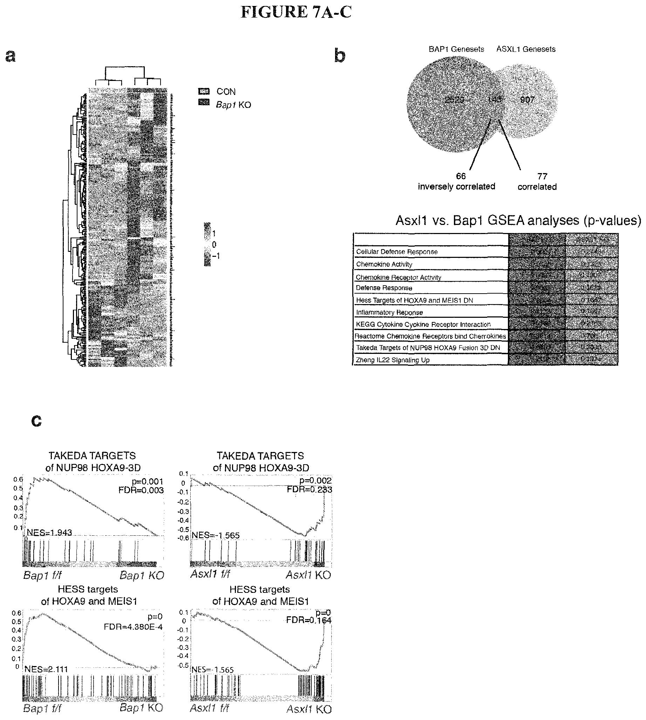

FIGURE 7A-C. *Bap1* and *Asxl1* loss results in opposite gene expression changes. (a) RNA-Seq data of differentially expressed genes in control versus *Bap1* KO mice granulocyte-macrophage progenitors (GMPs; Lin⁻c-Kit⁺Sca1⁻ CD34⁺ Fcγ⁺); cells analyzed with DESeq2 (cutoff p-value *p*<0.05). Heatmap indicates genes increasing (red) and decreasing (blue) in expression. (b) Number of positively and negatively enriched genesets from the *Bap1* KO and *Asxl1* KO GSEA analysis hitting an FDR<0.25 (top). Venn diagram depicting gene sets that are oppositely enriched in *Bap1* KO and *Asxl1* KO myeloid progenitors by RNA-Seq (bottom). (c) GSEA of oppositely enriched and statistically significant *HoxA* cluster gene sets in *Bap1* KO and *Asxl1* KO progenitor cells. *p*-values and FDR values are indicated.

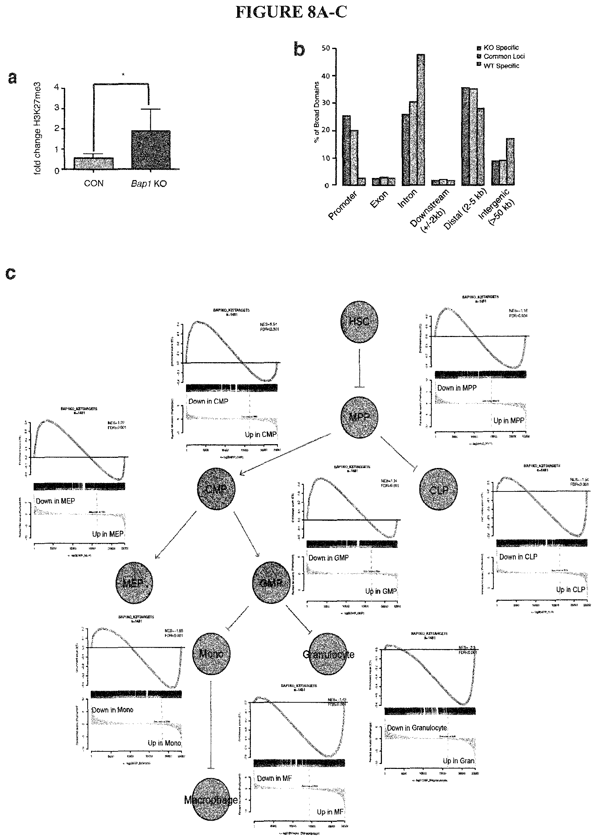

FIGURE 8A-C. Bap1 deletion enhances PRC2 activity. (a) ELISA of H3K27me3 normalized to total H3 in histones purified from bone marrow cells from *Bap1* KO and control mice. (b) Percentage of H3K27me3-broad domains called in relation to gene transcriptional start site (promoter, exon, intron, downstream (+/-2 kb), distal (2-5 kb), and intergeneic (>50 kb)). (c) Published RNA-Seq from sorted bone marrow populations (Lara-Astiaso et al., 2014) was analyzed and compared to genes that were differentially downregulated and marked with H3K27me3 following *Bap1* loss were analyzed using GSEA. Genes that were downregulated and marked by H3K27me3 were only correlated with the hematopoietic progenitor populations, suggesting that these may be the relevant target populations. These data are explanatory of the progenitor expansion that were seen in the *Bap1* KO mouse model.

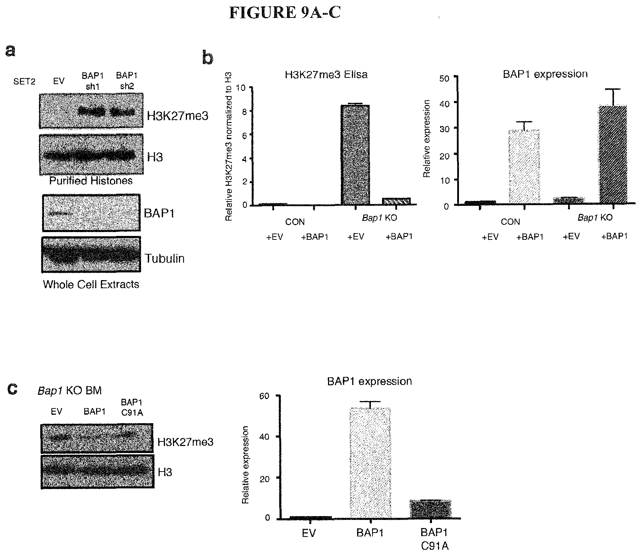

FIGURE 9A-C. *In vitro* BAP1 perturbations lead to changes in H3K27me3. (a) Western blot of SET2 cells transduced with two independent BAP1 shRNAs revealing H3K27me3 levels in purified histones and BAP1 knockdown from whole cell extract. (b) Methylcellulose assay with control and *Bap1* KO bone marrow cells. BAP1 cDNA constructs were reintroduced into control and Bap1 deleted cells. Histone ELISA assays were performed for H3K27me3. Quantitative qPCR to assess expression of BAP1 construct. (c) Reintroduction of BAP1 and deubiquitinase mutant BAP1 C91A in Bapl-deficient murine cells. Histone Western blots were performed for H3K27me3 and total H3. Quantitative qPCR to show levels of construct expression.

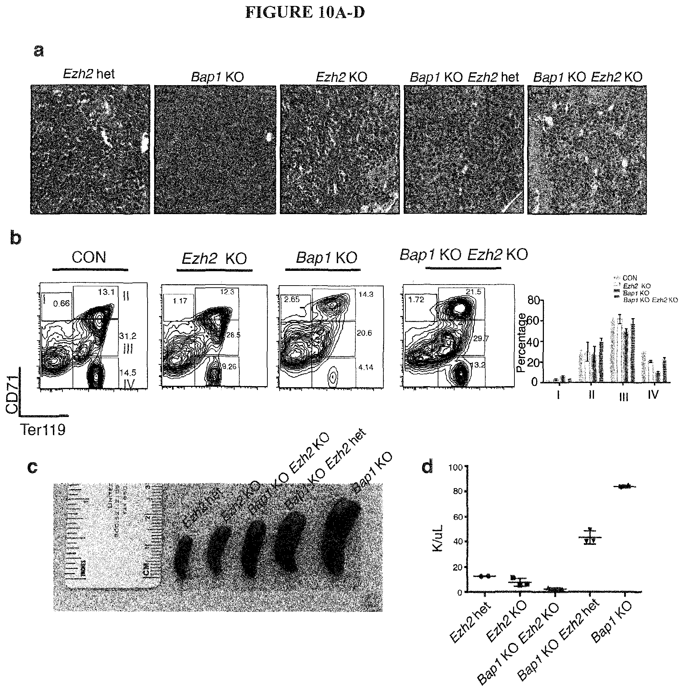

FIGURE 10A-D. Characterization of *Bap1*/*Ezh2* compound KO mice. (a) Bone marrow pathology for various *Bap1*/*Ezh2* genotypes. (b) Flow cytometric staining for erythroid cells in indicated genotypes (CD71, Ter119) and quantification. (I-IV) are indicative of stages of erythroid differentiation with I being the most immature and IV being the most mature. Quantitation of these phenotypes on the right of the representative flow plots. (c) Spleen sizes for indicated genotypes, 4 weeks post-pIpC. (d) White blood cell counts for indicated genotypes, 4 weeks post-pIpC.

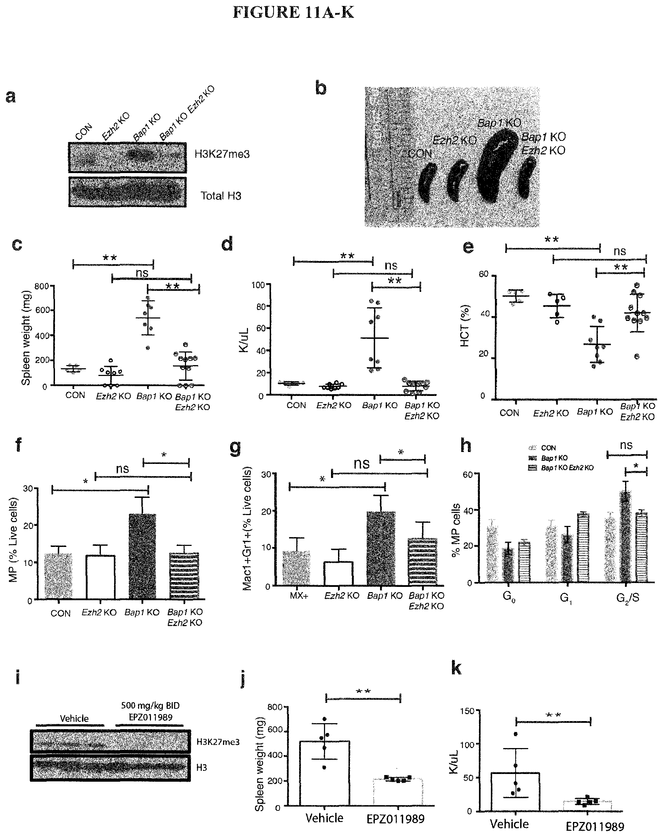

FIGURE 11A-K. Proliferation induced by *Bap1* deletion is rescued by loss of *Ezh2.* (a) Western blot of H3K27me3 levels in histones purified from bone marrow of *Bap1* KO, *Ezh2* KO, *Bap1*/*Ezh2* KO and control mice. (b) Representative images of spleens and (c) enumeration of spleen weight from the indicated genotypes of mice, 3 weeks post pIpC. (d) Peripheral white blood cell counts and (e) hematocrit percentages as quantified by a Hemavet. (f) Flow cytometric enumeration of myeloid progenitors (Lin⁻ c-Kit⁺ Sca1⁻) and (g) Mature myeloid cells (Mac1⁺Gr1⁺) (h) Cell cycle analyses in myeloid progenitors using Ki67 and DAPI stain (n=3/group) (i) Western blot for H3K27me3 levels in mice (n=5/group) treated with EPZ011989 twice a day at 500 mg/kg for 16 days. (j) Spleen weights and (k) white blood cell counts after treatment. Unless otherwise indicated, n=5/CON, *n=5*/*Ezh2* KO, *n=8*/*Bap1* KO, and n=11/*Bap1*/*Ezh2* KO, Statistics were calculated with Student t-test; *p<0.05, **p<0.005; ± SEM values reported.

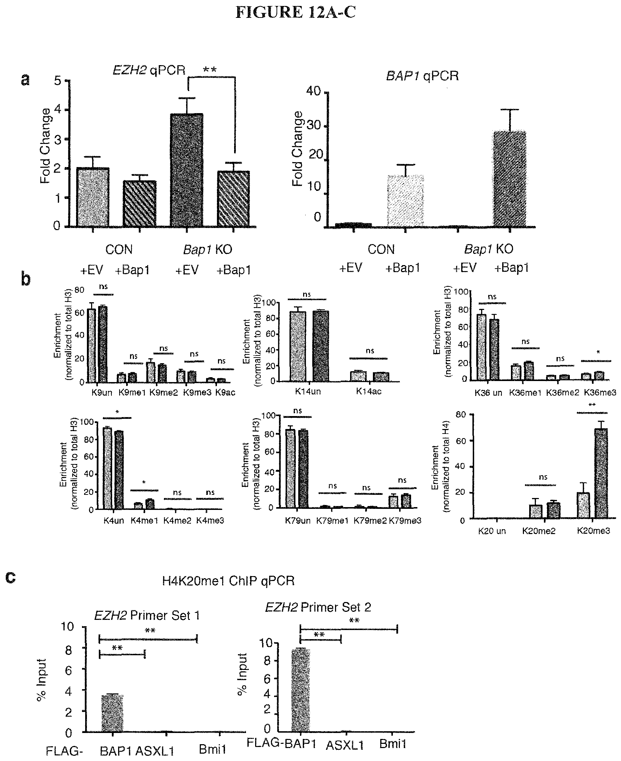

FIGURE 12A-C. Histone analyses *in Bap1* KO animals. (a) EZH2 transcription as assessed by qPCR in control and *Bap1* KO cells. Cells were either transduced with empty vector or a BAP1 overexpression construct. (b) Histone mass spectrometry in control and *Bap1* KO animals c-kit enriched bone marrow cells, n=2. (c) H4K20me1 ChIP-qPCR experiments in 293T cells that overexpress FLAG-tagged BAP1, ASXL1 and Bmi1.

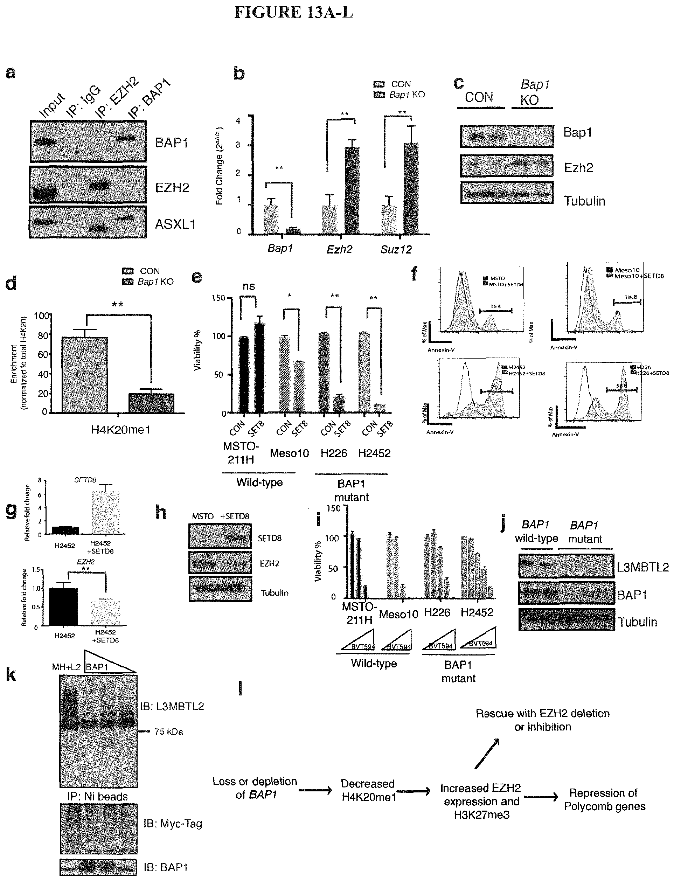

FIGURE 13A-L. BAP1 depletion leads to an increase in PRC2 component expression, increased H4K20me1 and deubiquitination of L3MBTL2. (a) Co-immunoprecipitation of endogenous EZH2 and BAP1 in SET2 cells followed by Western blot analysis (performed in presence of benzonase to inhibit interactions dependent on DNA). (b) *Bap1, Ezh2* and *Suz12* expression by qRT-PCR from sorted granulocyte-macrophage progenitor (GMP; Lin⁻c-Kit⁺Sca1⁻CD34⁺Fcγ⁺). (c) Western blot analysis of Bap1 and Ezh2 in bone marrow cells from *Bap1* KO and control mice. (d) H4K20me1 quantification from histone mass spectrometry experiments, (e) Cell viability as assessed by Cell Titer Glo viability assay and (f) Annexin V assays for SETD8 overexpression experiments in *BAP1* wild-type (MSTO-211H, Meso10) and mutant cell lines H226, deletion; H2452 catalytic mutation). (g) Quantitative qPCR for *SETD8* and *EZH2* in BAP1 mutant cells with SETD8 overexpression. (h) Western blot analysis for SETD8 and EZH2 in a BAP1 wild-type cell line. (i) Cell Titer Glo assay in cells treated with DMSO or 5, 10, 20 µM BVT594. (j) L3MBTL2 and BAP1 expression *in BAP1* wild-type (Met5a, JMN) and mutant mesothelioma cell lines (H226, H2452, H28). (k) 293T cells overexpressing Myc-His tagged ubiquitin and L3MBTL2 cDNA and varying levels of BAP1 (0, 5 µg, 2.5 µg, 1 µg). Co-immunoprecipitation experiments were conducted with Ni-beads and a series of stringent washes. (1) Model depicting the regulation of BAP1 leading to effects on chromatin and gene expression. Statistics were calculated with Student t-test; *p<0.05, **p<0.005; ± SEM values reported.

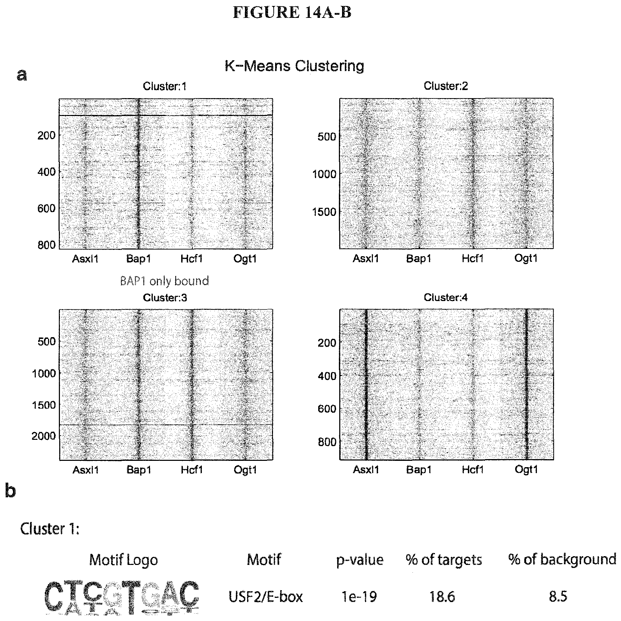

FIGURE 14A-B. Analysis of BAP1, ASXL1, HCF-1, and OGT binding. (a) K-means clustering analyses for BAP1, ASXL1, HCF-1, and OGT ChIP-Seq. (b) Homer de novo motif analyses in BAP1-bound clusters.

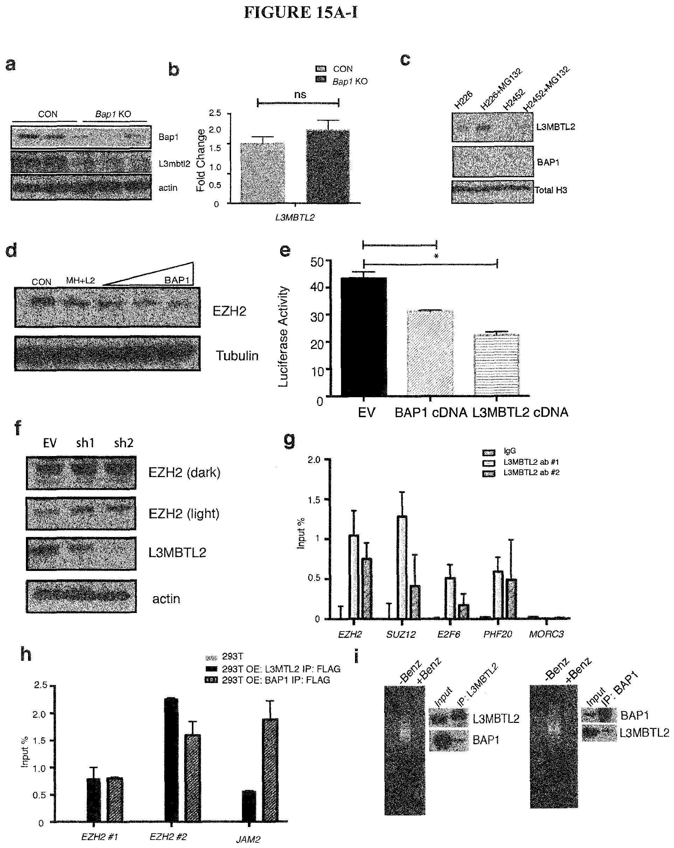

FIGURE 15A-I. L3MBTL2 and BAP1 co-regulate EZH2. (a) Expression of Bap1 and L3mblt2 in control and *Bap1* KO bone marrow cells. (b) Expression of *L3mbtl2* by qPCR in GMPs. (c) Western blot of H226 and H2452 cells treated with 25 uM MG132. Insoluble fractions were extracted using 2% SDS containing lysis buffer. (d) Expression of EZH2 in cell lines overexpressing L3MBTL2. (e) EZH2 promoter activity assay with a construct containing 1.9 kB of the EZH2 promoter and a Renilla control vector transiently transfected into 293T cells with either empty vector, a BAP1 or L3MBTL2 expression vector. Firefly luciferase activity was normalized to Renilla activity in each of these conditions. (f) Two independent hairpins were used to knockdown L3MBTL2 protein in SET2 cells. Western blot analyses were conducted on L3MBTL2, EZH2, and actin including short and long exposures. (g) ChIP for *L3MBTL2* followed by qPCR at the *EZH2, SUZ12, E2F6* (positive control), *PHF20* (positive control), and *MORC3* (negative control) loci in 293T cells. (h) Anti-FLAG ChIP followed by qPCR at the *EZH2* locus in 293T cells overexpressing FLAG-L3MBTL2 or FLAG-BAP1. JAM2 is a positive control. Compared to 293T cells without FLAG overexpression. (i) Western blot for L3MBTL2 and BAP1 following respective IPs in 293T cells. Agarose DNA gel included to show DNA digestion.

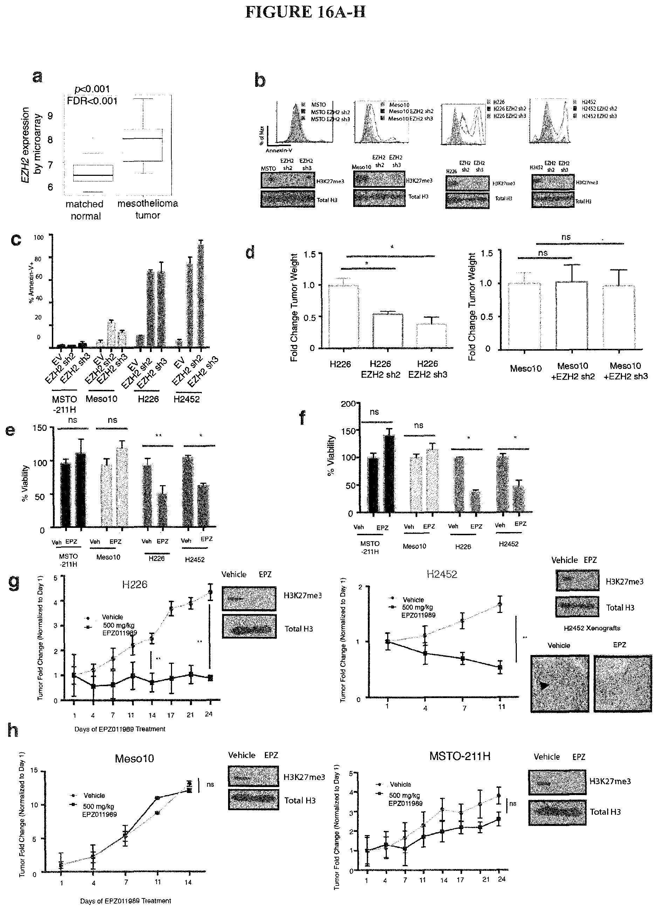

FIGURE 16A-H. *BAP1*-mutant mesothelioma cell lines and xenograft models are sensitive to EZH2 inhibition. (a) Expression of *EZH2* transcripts in TCGA mesothelioma patients compared to matched normals. (b) Annexin V assays in *BAP1* wild-type and mutant cell lines expressing either empty vector or hairpins targeting EZH2. (c) Quantitation of Annexin V experiments in mesothelioma cell lines (d) Tumor size of Meso10 and H226 cell lines expressing EZH2 hairpins implanted into NOD-SCID mice, n=6/group. (e) 2D Cell Titer Glo viability assays after 2 week treatment with EPZ011989 at 1.25 µM. (f) 3D Cell Titer Glo viability assays after 3 weeks EPZ011989 treatment at 1.25 µM. (g) Tumor size formation from *BAP1* mutant (MSTO and Meso10) or (h) wild-type cells (H226 and H2452) implanted into NOD-SCID mice and treated with either vehicle or 500 mg/kg BID EPZ011989. Tumors were measured 3X weekly, n=6/group. Target inhibition was assessed by histone western blots in extracted tumors (shown in respective figures). Lung pathology of H2452 cells with vehicle and EPZ011989 treatment. Arrow indicates bulk metastasized tumor. Statistics were calculated with Student t-test; *p<0.05, **p<0.005; ± SEM values reported.

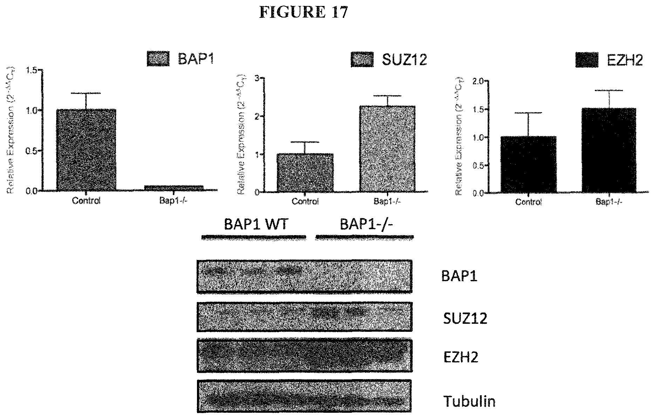

FIGURE 17. PRC2 component expression was increased in sorted populations and in whole bone marrow.

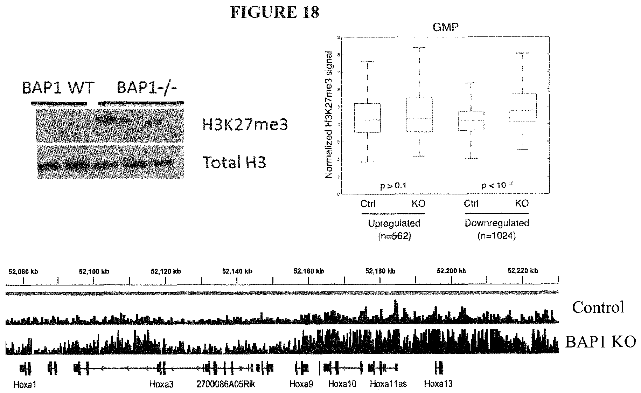

FIGURE 18. H3K27me3 was locally and globally increased in BAP1 KO mice. Histone methylation in the *BAP1* KO animals was assessed by conducting acid extraction followed by western blot on control and *BAP1* KO bone marrow. Chromatin Immunoprecipitation Sequencing (ChIP-Seq) was also completed on cKit enriched bone marrow. Overlaying ChIP-Seq with RNA-Sequencing data demonstrated that genes downregulated in the *BAP1* KO mice were increasingly marked with H3K27me3. In *BAP1* KO mice, H3K27me3 was observed to be increased at EZH2 target genes such as the HOXA locus.

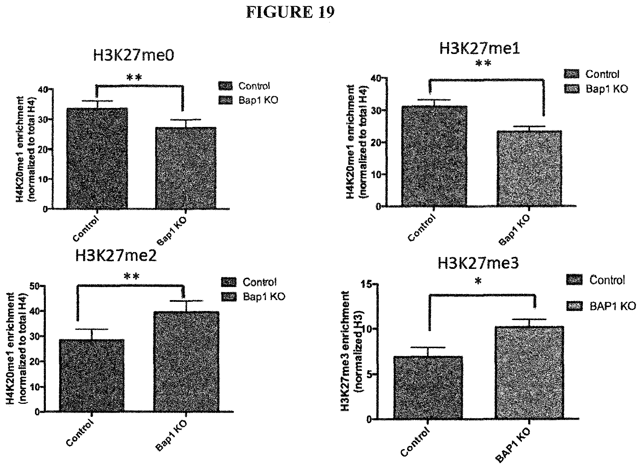

FIGURE 19. Upregulation of H3K27me2/3 *in BAP1* KO cells occurred at the expense of H3K27me0/1.

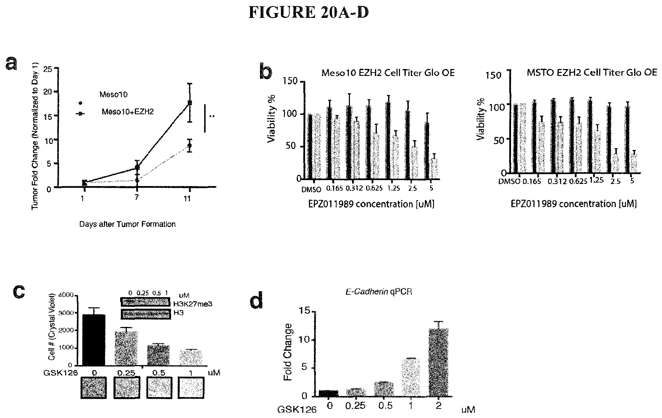

FIGURE 20A-D. BAP1 mutant cell lines are most sensitive to EZH2 inhibition. (a) Meso10 cell line overexpressing EZH2 increasingly proliferated after injection into the flank of NOD-SCID mice. (b) EZH2 was overexpressed in MSTO-211H and Meso10 cell lines. The cell lines became increasingly sensitive to EZP011989 with EZH2 overexpression. (c) *BAP1*-mutant cells became less invasive when treated with the EZH2 inhibitor GSK126. (d) E-Cadherin expression increased in the cell line H226 following treatment with GSK126.

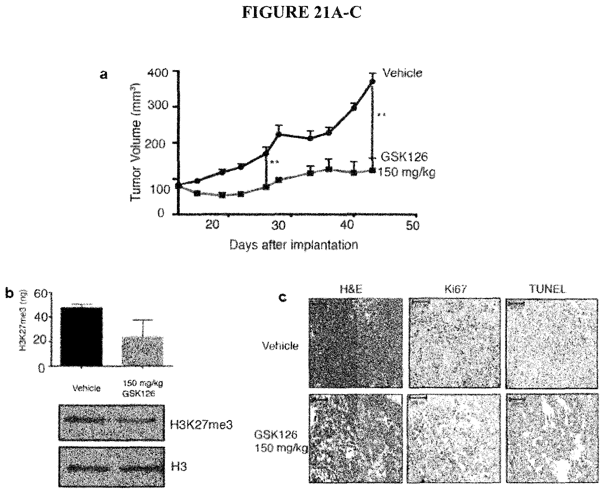

FIGURE 21A-C. *BAP1*-mutant mesothelioma cell lines and xenograft models are sensitive to EZH2 inhibition. (a) Tumor volume of H2452 xenografts treated daily with GSK126 at 150 mg/kg or vehicle (n=10 mice per group). 5 mice from each group were euthanized following 16 days of treatment to assess H3K27me3 depletion. The remaining mice were treated for the remainder of the trial. (b) Histone ELISA and Western blot analysis of H3K27me3 levels in tumors from in vivo treated mice after 16 days treatment. (c) H&E staining, Ki67 staining and TUNEL staining of tumors extracted from vehicle treated and GSK126 treated mice, 10X magnification.

### 5. DETAILED DESCRIPTION

For clarity and not by way of limitation the detailed description of the present disclosure is divided into the following subsections:
(i) BAP1 biomarkers;
(ii) EZH2 inhibitors;
(iii) cancer targets;
(iv) biomarker detection;
(v) methods of use; and
(vi) kits.

### 5.1 BAP1 BIOMARKERS

The term "biomarker" as used herein, includes nucleic acids and proteins that are related to the activity level of BRCA1 associated protein-1, denoted as BAP1 herein, in a subject.

A "patient" or "subject," as used interchangeably herein, refers to a human or a non-human subject. Non-limiting examples of non-human subjects include non-human primates, dogs, cats, mice, rats, guinea pigs, rabbits, pigs, fowl, horses, cows, goats, sheep, cetaceans, etc.

In certain non-limiting embodiments, a disclosed BAP1 biomarker may be a nucleic acid. For example, but not by way of limitation, the biomarker can be a deoxyribonucleic acid (DNA) or a ribonucleic acid (RNA), *e.g.,* mRNA.

In certain, non-limiting embodiments, a BAP1 nucleic acid biomarker may be a human *BAP1* nucleic acid having the sequence as set forth in NCBI database accession no. NG_031859.1 or NM_004656, or a nucleic acid encoding a BAP1 protein molecule that has the amino acid sequence as set forth in NCBI database accession no. NP_004647.

In a specific, non-limiting embodiment, a BAP1 nucleic acid biomarker may be a mouse *BAP1* nucleic acid having the sequence as set forth in NCBI database accession no. NM_027088, or a nucleic acid encoding a BAP1 protein molecule that has the amino acid sequence as set forth in NCBI database accession NP_081364.1.

In a specific, non-limiting embodiment, a BAP1 nucleic acid biomarker may be a rat *BAP1* nucleic acid having the sequence as set forth in NCBI database accession no. NM_001107292.1, or a nucleic acid encoding a BAP1 protein molecule that has the amino acid sequence as set forth in NCBI database accession NP_001100762.1.

In certain non-limiting embodiments, a BAP1 biomarker may be a protein.

In a specific, non-limiting embodiment, a BAP1 protein biomarker may be a human BAP1 protein having the amino acid sequence as set forth in NCBI database accession no. NP_004647.

In a specific, non-limiting embodiment, a BAP1 protein biomarker may be a mouse BAP1 protein having the amino acid sequence as set forth in NCBI database accession no. NP_081364.1.

In a specific, non-limiting embodiment, a BAP1 protein biomarker may be a rat BAP1 protein having the amino acid sequence as set forth in NCBI database accession no. NP_001100762.1.

In certain embodiments, the level of the BAP1 biomarker is compared to a reference control level. A "reference control level" or "reference control expression level" of BAP1, as used interchangeably herein, may, for example, be established using a reference control sample. Non-limiting examples of reference control samples include normal and/or healthy cells that have wild-type BAP1 activity. In certain embodiments, a reference control level of BAP1 may, for example, be established using normal cells, *e.g*., benign cells, located adjacent to the tumor in a patient.

In certain, non-limiting embodiments of the present disclosure, a level of a BAP1 biomarker may be evaluated by evaluating BAP1 function, where the BAP1 expression level is directly proportional to the level of BAP1 function. In one non-limiting example, the function of BAP1 can be downregulation of EZH2 expression (e.g., EZH2 protein expression or nucleic acid expression). For example, and not by way of limitation, the level of a BAP1 biomarker may be determined by determining the expression level of EZH2 in a cancer cell of a subject compared to an EZH2 reference control level. In certain embodiments, an EZH2 reference control level can be established using normal and/or healthy cells that have wild-type and/or normal EZH2 activity and/or normal BAP1 activity. In certain non-limiting embodiments, EZH2 may be a human *EZH2* nucleic acid having the sequence as set forth in NCBI database accession no. NG_032043.1; NM_004456.4; NM_001203249.1; NM_152998.2; NM_001203247.1 and/or NM_001203248.1, or a nucleic acid encoding a EZH2 protein molecule that has the amino acid sequence set forth in NCBI database accession no. NP_001190176.1; NP_001190177.1; NP_001190178.1; NP_004447.2; and/or NP_694543.1. In a specific, non-limiting embodiment, EZH2 may be a human EZH2 protein having the amino acid sequence as set forth in NCBI database accession no. NP_001190176.1; NP_001190177.1; NP_001190178.1; NP_004447.2; and/or NP_694543.1.

In one non-limiting example, the function of BAP1 can be regulation of SUZ12 expression (*e.g*., SUZ12 protein expression or nucleic acid expression). In certain non-limiting embodiments, the level of a BAP1 biomarker may be determined by determining the expression level of SUZ12 in a cancer cell of a subject compared to a SUZ12 reference control level. In certain embodiments, a SUZ12 reference control level can be established using normal and/or healthy cells that have wild-type and/or normal SUZ12 activity and/or normal BAP1 activity. In certain non-limiting embodiments, SUZ12 may be a human *SUZ12* nucleic acid having the sequence as set forth in NCBI database accession no. NM_015355.2, or a nucleic acid encoding a SUZ12 protein molecule that has the amino acid sequence set forth in NCBI database accession no. NP_056170.2. In a specific, non-limiting embodiment, SUZ12 may be a human SUZ12 protein having the amino acid sequence as set forth in NCBI database accession no. NP_056170.2.

Where comparisons to reference control expression levels are referred to herein, the biomarker is assessed relative to the reference control expression level within the same species. For example, a human BAP1 biomarker expression level and/or presence are compared with a human BAP1 reference control level.

In particular non-limiting embodiments, the absence and/or a reduced expression of a BAP1 biomarker means the detection of less than about 90%, less than about 80%, less than about 70%, less than about 60%, less than about 50%, less than about 40%, less than about 30% expression relative to the reference control level.

### 5.2 EZH2 INHIBITORS

Non-limiting examples of EZH2 inhibitors include compounds, molecules, chemicals, polypeptides, proteins that inhibit and/or reduce the expression and/or activity of EZH2. Additional non-limiting examples of EZH2 inhibitors include S-adenosyl-methionine-competitive small molecule inhibitors. In particular non-limiting embodiments, the EZH2 inhibitor is derived from tetramethylpiperidinyl compounds. Further non-limiting examples include UNC1999, 3-Deazaneplanocin A (DZNep), EI1, EPZ-5676, EPZ-6438, GSK343, EPZ005687, EPZ011989 and GSK126.

Further non-limiting examples of EZH2 inhibitors are described in Garapaty-Rao et al., Chemistry and Biology, 20: pp. 1-11 (2013), PCT Patent Application Nos. WO 2013/138361, WO 2013/049770 and WO 2003/070887, and US Patent Nos. US 9,334,527, US 8,895,245 and US 9,688,665 and US Patent Application No. 2011/0251216.

Further non-limiting examples of EZH2 inhibitors include ribozymes, antisense oligonucleotides, shRNA molecules and siRNA molecules that specifically inhibit the expression or activity of EZH2. One non-limiting example of an EZH2 inhibitor comprises an antisense, shRNA, or siRNA nucleic acid sequence homologous to at least a portion of a EZH2 nucleic acid sequence, wherein the homology of the portion relative to the EZH2 sequence is at least about 75 or at least about 80 or at least about 85 or at least about 90 or at least about 95 or at least about 98 percent, where percent homology can be determined by, for example, BLAST or FASTA software. In certain non-limiting embodiments, the complementary portion may constitute at least 10 nucleotides or at least 15 nucleotides or at least 20 nucleotides or at least 25 nucleotides or at least 30 nucleotides and the antisense nucleic acid, shRNA or siRNA molecules may be up to 15 or up to 20 or up to 25 or up to 30 or up to 35 or up to 40 or up to 45 or up to 50 or up to 75 or up to 100 nucleotides in length. Antisense, shRNA or siRNA molecules may comprise DNA or atypical or non-naturally occurring residues, for example, but not limited to, phosphorothioate residues.

In certain non-limiting embodiments, the EZH2 inhibitor can be used alone or in combination with one or more anti-cancer agents. An anti-cancer agent can be any molecule, compound chemical or composition that has an anti-cancer effect. Anti-cancer agents include, but are not limited to, chemotherapeutic agents, radiotherapeutic agents, cytokines, anti-angiogenic agents, apoptosis-inducing agents or anti-cancer immunotoxins, such as antibodies. "In combination with" means that the EZH2 inhibitor and the one or more anti-cancer agents are administered to a subject as part of a treatment regimen or plan. These terms do not require that the EZH2 inhibitor and one or more anti-cancer agents are physically combined prior to administration nor that they be administered over the same time frame.

### 5.3 CANCER TARGETS

Cancers that are subject to the presently disclosed subject matter include malignant mesotheliomas, uveal melanomas, renal cell carcinoma, cutaneous melanomas, lung cancer, breast cancer, ovarian cancer, non-melanoma skin cancer, meningioma, cholangiocarcinoma, leiomysarcoma, neuroendocrine tumors, pancreatic cancer, paraganglioma, malignant fibrous histiocytoma, myelodysplastic syndromes, acute myeloid leukemia, melanocytic BAP1-mutated atypical intradermal tumors (MBAITs) and bladder cancer.

### 5.4 BIOMARKER DETECTION

Methods for qualitatively and quantitatively detecting and/or determining the expression level of a BAP1 nucleic acid biomarker, an EZH2 nucleic acid, an L3MBTL2 nucleic acid or a SUZ12 nucleic acid include, but are not limited to polymerase chain reaction (PCR), including conventional, qPCR and digital PCR, *in situ* hybridization (for example, but not limited to Fluorescent In Situ Hybridization ("FISH")), gel electrophoresis, sequencing and sequence analysis, microarray analysis and other techniques known in the art.

In certain embodiments, the method of detection can be real-time PCR (RT-PCR), quantitative PCR, fluorescent PCR, RT-MSP (RT methylation specific polymerase chain reaction), PicoGreen™ (Molecular Probes, Eugene, OR) detection of DNA, radioimmunoassay or direct radio-labeling of DNA. For example, but not by way of limitation, a nucleic acid biomarker can be reversed transcribed into cDNA followed by polymerase chain reaction (RT-PCR); or, a single enzyme can be used for both steps as described in U.S. Pat. No. 5,322,770, or the biomarker can be reversed transcribed into cDNA followed by symmetric gap ligase chain reaction (RT-AGLCR) as described by R. L. Marshall, et al., PCR Methods and Applications 4: 80-84 (1994). Non-limiting examples of primers for use in the disclosed methods are shown in Table 1.

In certain embodiments, quantitative real-time polymerase chain reaction (qRT-PCR) is used to evaluate mRNA levels of biomarker. The levels of a biomarker and a control mRNA can be quantitated in cancer tissue or cells and adjacent benign tissues. In certain embodiments, the levels of one or more biomarkers can be quantitated in a biological sample.

In a non-limiting embodiment, the method of detection of the present disclosure can be carried out without relying on amplification, *e.g*., without generating any copy or duplication of a target sequence, without involvement of any polymerase, or without the need for any thermal cycling. In certain embodiments, detection of the present disclosure can be performed using the principles set forth in the QuantiGene™ method described in U.S. Patent No. 7709198, filed Jun. 19, 2006.

In certain embodiments, *in situ* hybridization visualization can be employed, where a radioactively labeled antisense RNA probe is hybridized with a thin section of a biological sample, *e.g.,* a biopsy sample, washed, cleaved with RNase and exposed to a sensitive emulsion for autoradiography. The samples can be stained with haematoxylin to demonstrate the histological composition of the sample, and dark field imaging with a suitable light filter shows the developed emulsion. Non-radioactive labels such as digoxigenin can also be used.

In certain non-limiting embodiments, evaluation of nucleic acid biomarker expression can be performed by fluorescent *in situ* hybridization (FISH). FISH is a technique that can directly identify a specific region of DNA or RNA in a cell and therefore enables visual determination of the biomarker expression in tissue samples. The FISH method has the advantages of a more objective scoring system and the presence of a built-in internal control consisting of the biomarker gene signals present in all non-neoplastic cells in the same sample. FISH is a direct *in situ* technique that can be relatively rapid and sensitive, and can also be automated. Immunohistochemistry can be combined with a FISH method when the expression level of the biomarker is difficult to determine by FISH alone.

In certain embodiments, expression of a nucleic acid biomarker can be detected on a DNA array, chip or a microarray. Oligonucleotides corresponding to the biomarker(s) are immobilized on a chip which is then hybridized with labeled nucleic acids of a biological sample, *e.g*., tumor sample, obtained from a subject. Positive hybridization signal is obtained with the sample containing biomarker transcripts. Methods of preparing DNA arrays and their use are well known in the art. (*See,* for example, U.S. Patent Nos. 6,618,6796; 6,379,897; 6,664,377; 6,451,536; 548,257; U.S. Patent No. 6989262 and Schena et al. 1995 Science 20:467-470; Gerhold et al. 1999 Trends in Biochem. Sci. 24, 168-173; and Lennon et al. 2000 Drug discovery Today 5: 59-65. Serial Analysis of Gene Expression (SAGE) can also be performed (*See,* for example, U.S. Patent Application No. 20030215858).

In certain embodiments, to monitor a nucleic acid biomarker, *e.g*., BAP1 mRNA, expression levels, mRNA can be extracted from the biological sample to be tested, reverse transcribed and fluorescent-labeled cDNA probes can be generated. The labeled cDNA probes can then be applied to microarrays capable of hybridizing to a biomarker, allowing hybridization of the probe to microarray and scanning the slides to measure fluorescence intensity. This intensity correlates with the hybridization intensity and expression levels of the biomarker.

Types of probes for detection of nucleic acid biomarkers include cDNA, riboprobes, synthetic oligonucleotides and genomic probes. The type of probe used will generally be dictated by the particular situation, such as riboprobes for in situ hybridization, and cDNA for Northern blotting, for example. In certain non-limiting embodiments, the probe is directed to nucleotide regions unique to the particular biomarker RNA. The probes can be as short as is required to differentially recognize the particular biomarker mRNA transcripts, and can be as short as, for example, 15 bases. Probes of at least 17 bases, 18 bases and 20 bases can also be used. In certain embodiments, the primers and probes hybridize specifically under stringent conditions to a nucleic acid fragment having the nucleotide sequence corresponding to the target gene. As herein used, the term "stringent conditions" means hybridization will occur only if there is at least 95% or at least 97% identity between the sequences.

The form of labeling of the probes can be any that is appropriate, such as the use of radioisotopes, for example, ³²P and ³⁵S, or fluorophores. Labeling with radioisotopes can be achieved, whether the probe is synthesized chemically or biologically, by the use of suitably labeled bases.

Methods for detecting and/or determining the level of a protein biomarker, *e.g*., a BAP1 protein biomarker, EZH2 protein, L3MBTL2 protein or SUZ12 protein, are well known to those skilled in the art, and include, but are not limited to, mass spectrometry techniques, 1-D or 2-D gel-based analysis systems, chromatography, enzyme linked immunosorbent assays (ELISAs), radioimmunoassays (RIA), enzyme immunoassays (EIA), Western Blotting, immunoprecipitation and immunohistochemistry. These methods use antibodies, or antibody equivalents, to detect protein, or use biophysical techniques. Antibody arrays or protein chips can also be employed, *see, for example,* U.S. Patent Application No. 2003/0013208; and U.S. Pat. Nos. US6,329,209, US7,429,466, US7,112,408 and US6,365,418.

In certain non-limiting embodiments, a detection method for measuring protein biomarker expression includes the steps of: contacting a biological sample, *e.g.,* a tissue sample, with an antibody or variant (*e.g.,* fragment) thereof, which selectively binds the biomarker, and detecting whether the antibody or variant thereof is bound to the sample. The method can further include contacting the sample with a second antibody, *e.g*., a labeled antibody. The method can further include one or more washing steps, *e.g.,* to remove one or more reagents.

In certain non-limiting embodiments, Western blotting can be used for detecting and quantitating biomarker protein expression levels. Cells can be homogenized in lysis buffer to form a lysate and then subjected to SDS-PAGE and blotting to a membrane, such as a nitrocellulose filter. Antibodies (unlabeled) can then brought into contact with the membrane and assayed by a secondary immunological reagent, such as labeled protein A or anti-immunoglobulin (suitable labels including ¹²⁵I, horseradish peroxidase and alkaline phosphatase). Chromatographic detection can also be used. In certain embodiments, immunodetection can be performed with antibody to a biomarker using the enhanced chemiluminescence system (e.g., from PerkinElmer Life Sciences, Boston, Mass.). The membrane can then be stripped and re-blotted with a control antibody specific to a control protein, *e.g*., actin.

Immunohistochemistry can be used to detect the expression and/or presence of a biomarker, *e.g*., in a biopsy sample. A suitable antibody can be brought into contact with, for example, a thin layer of cells, followed by washing to remove unbound antibody, and then contacted with a second, labeled, antibody. Labeling can be by fluorescent markers, enzymes, such as peroxidase, avidin or radiolabeling. The assay can be scored visually, using microscopy, and the results can be quantitated. Machine-based or autoimaging systems can also be used to measure immunostaining results for the biomarker.

Various automated sample processing, scanning and analysis systems suitable for use with immunohistochemistry are available in the art. Such systems can include automated staining (see, *e.g.,* the Benchmark system, Ventana Medical Systems, Inc.) and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples. *See, e.g.,* the CAS-200 system (Becton, Dickinson & Co.).

Labeled antibodies against biomarkers can also be used for imaging purposes, for example, to detect the presence of a biomarker in cells of a subject. Suitable labels include radioisotopes, iodine (¹²⁵I, ¹²¹I), carbon (¹⁴C), sulphur (³⁵S), tritium (³H), indium (¹¹²In), and technetium (99mTc), fluorescent labels, such as fluorescein and rhodamine, and biotin. Immunoenzymatic interactions can be visualized using different enzymes such as peroxidase, alkaline phosphatase, or different chromogens such as DAB, AEC or Fast Red. The labeled antibody or antibody fragment will preferentially accumulate at the location of cells which contain a biomarker. The labeled antibody or variant thereof, *e.g*., antibody fragment, can then be detected using known techniques.

Antibodies include any antibody, whether natural or synthetic, full length or a fragment thereof, monoclonal or polyclonal, that binds sufficiently strongly and specifically to the biomarker to be detected. An antibody can have a K_{d} of at most about 10⁻⁶M, 10⁻⁷M, 10⁻⁸M, 10⁻⁹M, 10⁻¹⁰M, 10⁻¹¹M and 10⁻¹²M. The phrase "specifically binds" refers to binding of, for example, an antibody to an epitope or antigen or antigenic determinant in such a manner that binding can be displaced or competed with a second preparation of identical or similar epitope, antigen or antigenic determinant.

Antibodies, and derivatives thereof, that can be used encompass polyclonal or monoclonal antibodies, synthetic and engineered antibodies, chimeric, human, humanized, primatized (CDR-grafted), veneered or single-chain antibodies, phase produced antibodies (*e.g*., from phage display libraries), as well as functional binding fragments, of antibodies. For example, antibody fragments capable of binding to a biomarker, or portions thereof, including, but not limited to, Fv, Fab, Fab' and F(ab')₂ fragments, can be used. Such fragments can be produced by enzymatic cleavage or by recombinant techniques. Non-limiting examples of commercially available BAP1 antibodies include SC-8132, SC-48386, SC-13576, SC-28236, SC-8133 and SC-28383 from Santa Cruz Biotechnology (Santa Cruz, CA), Ab167250 from Abcam (Cambridge, England) and HPA028814 from Sigma-Aldrich (St. Louis, MO). Non-limiting examples of commercially available EZH2 antibodies include Cat Nos. 39933, 39875 and 39901 from Active Motif (Carlsbad, CA), 07-689 from Millipore (Billerica, MA) and PA1-46476 and PA5-24594 from Thermo Fisher Scientific (Waltham, MA). A non-limiting example of a commercially available SUZ12 antibody includes Ab12073 from Abcam. A non-limiting example of a commercially available L3MBTL2 antibody includes 39569 from Active Motif.

In certain non-limiting embodiments, agents that specifically bind to a polypeptide other than antibodies are used, such as peptides. Peptides that specifically bind can be identified by any means known in the art, *e.g.,* peptide phage display libraries. Generally, an agent that is capable of detecting a biomarker polypeptide, such that the presence of a biomarker is detected and/or quantitated, can be used. As defined herein, an "agent" refers to a substance that is capable of identifying or detecting a biomarker in a biological sample (e.g., identifies or detects the mRNA of a biomarker, the DNA of a biomarker, the protein of a biomarker).

In addition, a biomarker can be detected using Mass Spectrometry such as MALDI/TOF (time-of-flight), SELDI/TOF, liquid chromatography-mass spectrometry (LC-MS), gas chromatography-mass spectrometry (GC-MS), high performance liquid chromatography-mass spectrometry (HPLC-MS), capillary electrophoresis-mass spectrometry, nuclear magnetic resonance spectrometry, or tandem mass spectrometry (*e.g.,* MS/MS, MS/MS/MS, ESI-MS/MS, etc.). *See, for example,* U.S. Patent Application Nos. 2003/0199001 and 2003/0077616 and U.S. Patent No. 8,068,987.

Mass spectrometry methods are well known in the art and have been used to quantify and/or identify biomolecules, such as proteins (*see, e.g.,* Li et al. (2000) Tibtech 18:151-160; Rowley et al. (2000) Methods 20: 383-397; and Kuster and Mann (1998) Curr. Opin. Structural Biol. 8: 393-400). Further, mass spectrometric techniques have been developed that permit at least partial de novo sequencing of isolated proteins. Chait et al., Science 262:89-92 (1993); Keough et al., Proc. Natl. Acad. Sci. USA. 96:7131-6 (1999); reviewed in Bergman, EXS 88:133-44 (2000).

Detection of the presence of a biomarker or other substances will typically involve detection of signal intensity. This, in turn, can reflect the quantity and character of a polypeptide bound to the substrate. For example, in certain embodiments, the signal strength of peak values from spectra of a first sample and a second sample can be compared (e.g., visually or by computer analysis), to determine the relative amounts of a particular biomarker. Software programs such as the Biomarker Wizard program (Ciphergen Biosystems, Inc., Fremont, Calif.) can be used to aid in analyzing mass spectra.

Additional methods for determining nucleic acid and/or protein biomarker expression in samples are described, for example, in U.S. Pat. No. 6,271,002; U.S. Patent No. 6,218, 122; U.S. Patent No. 6,218, 114; and U.S. Patent No. 6,004,755; and in Wang et al, J. Clin. Oncol., 22(9): 1564-1671 (2004); and Schena et al, Science, 270:467-470 (1995).

### 5.5 METHODS OF USE

In certain non-limiting embodiments, the present disclosure provides for methods of determining whether an anti-cancer effect is likely to be produced in a cancer by an EZH2 inhibitor, comprising, determining the presence, absence and/or expression level of a BAP1 biomarker, *e.g*., a BAP1 nucleic acid and/or protein biomarker. Methods for determining the presence, absence and/or expression levels of a BAP1 biomarker are set forth in section 5.4 above. Cancers suitable for treatment are described above in section 5.3. EZH2 inhibitors are described above in section 5.2.

In certain embodiments, the present disclosure provides for a method of producing an anti-cancer effect in a cancer, comprising determining whether cells of the cancer contain a BAP1 biomarker, where if the BAP1 biomarker is absent and/or expressed at lower levels in the cancer, as compared to a reference control level, administering a therapeutically effective amount of an EZH2 inhibitor to produce an anti-cancer effect. Alternatively, if the BAP1 biomarker is found to be expressed at the same or higher levels relative to a reference control level, then an alternative therapy with an agent that is not an EZH2 inhibitor is administered.

A "therapeutically effective amount" refers to an amount that is able to achieve one or more of an anticancer effect, prolongation of survival and/or prolongation of period until relapse.

An "anti-cancer effect" refers to one or more of a reduction in aggregate cancer cell mass, a reduction in cancer cell growth rate, a reduction in cancer cell proliferation, a reduction in tumor mass, a reduction in tumor volume, a reduction in tumor cell proliferation, a reduction in tumor growth rate and/or a reduction in tumor metastasis. In certain embodiments, an anti-cancer effect can refer to a complete response, a partial response, a stable disease (without progression or relapse), a response with a later relapse or progression-free survival in a patient diagnosed with cancer.

In certain embodiments, the present disclosure provides for a method of producing an anti-cancer effect in a cancer, comprising determining the expression level of a BAP1 biomarker in one or more cells of the cancer, where if the BAP1 protein biomarker is absent and/or expressed at lower levels in the cells, as compared to a reference control level, then administering a therapeutically effective amount of an EZH2 inhibitor to produce an anti-cancer effect. In certain embodiments, the reference control is the level of BAP1 in normal cells, *e.g.,* benign cells, located adjacent to the cancer.

In certain non-limiting embodiments, the level of BAP1 in the cancer sample and/or the reference control sample can be normalized against a nucleic acid and/or protein present in both samples, *e.g.,* a reference protein or nucleic acid such as a housekeeping gene and/or protein, to allow comparison. For example, and not by way of limitation, the reference protein or nucleic acid can be actin or tubulin.

In certain non-limiting embodiments, the present disclosure provides a method of predicting the sensitivity of a cancer in a patient to an EZH2 inhibitor, comprising, obtaining a sample of the cancer from the patient and determining the expression level of a BAP1 protein biomarker in the cells comprising the sample, where if the BAP1 protein biomarker is absent or reduced in expression compared to a reference control level, then the cancer is predicted to be sensitive to the EZH2 inhibitor. In certain embodiments, if the cancer of a patient is predicted to be sensitive to the EZH2 inhibitor, the patient can then be treated with an EZH2 inhibitor. In certain embodiments, if the cancer of a patient is predicted to be insensitive to the EZH2 inhibitor, then the patient can be treated with an agent that is not an EZH2 inhibitor.

In certain non-limiting embodiments, the present disclosure provides a method for treating a subject having a cancer. For example, and not by way of limitation, the method comprises obtaining a sample of the cancer from the subject, and determining, in the sample, the expression level of a BAP1 biomarker, where if the BAP1 biomarker is absent or expressed at a lower level than a BAP1 reference control level, then initiating treatment of the subject with a therapeutically effective amount of an EZH2 inhibitor. Alternatively, if the BAP1 biomarker is found to be expressed at the same or higher levels relative to a reference control level, then the subject may be treated with an agent that is not an EZH2 inhibitor.

In certain embodiments, the methods of the present disclosure may further comprise detecting the expression level of EZH2, L3MBTL2 and/or SUZ12 in the sample. For example, but not by way of limitation, the mRNA and/or protein expression levels of EZH2, L3MBTL2 and/or SUZ12 can be detected. In certain embodiments, a method for treating a subject having a cancer, comprising, obtaining a sample of the cancer from the subject, and determining, in the sample, the expression level of a BAP1 biomarker and the expression level of EZH2, L3MBTL2 and/or SUZ12, where if the BAP1 biomarker is absent or expressed at a lower level than a BAP1 reference control level and the expression of EZH2 and/or SUZ12 is increased compared to an EZH2 and/or SUZ12 reference control level (and/or the expression of L3MBTL2 is decreased compared to a L3MBTL2 reference control level), then initiating treatment of the subject with a therapeutically effective amount of an EZH2 inhibitor. In certain embodiments, a sample may be collected before and after treatment with an EZH2 inhibitor and the EZH2 expression levels of the samples can be compared.

In certain non-limiting embodiments, a sample includes, but is not limited to, cells in culture, cell supernatants, cell lysates, serum, blood plasma, biological fluid (*e.g.,* blood, plasma, serum, stool, urine, lymphatic fluid, ascites, ductal lavage, nipple aspirate, saliva, broncho-alveolar lavage, tears and cerebrospinal fluid) and tissue samples. The source of the sample may be solid tissue (e.g., from a fresh, frozen, and/or preserved organ or tumor sample, tissue sample, biopsy, or aspirate), blood or any blood constituents, bodily fluids (such as, *e.g.,* urine, lymph, cerebral spinal fluid, amniotic fluid, peritoneal fluid, or interstitial fluid), or cells from the individual, including circulating tumor cells. In certain non-limiting embodiments, the sample is obtained from a tumor. In certain embodiments, the sample may be a "clinical sample," which is a sample derived from a patient.

### 5.6 KITS

In certain non-limiting embodiments, the present disclosure provides for a kit for determining whether an anti-cancer effect is likely to be produced in a cancer by an EZH2 inhibitor, comprising a means for detecting a BAP1 biomarker, as set forth in the preceding sections. Said kit may further include instructions or supporting material that describe the use of the kit to determine whether an anti-cancer effect is likely to be produced in a cancer by an EZH2 inhibitor and/or reference to a website or publication describing same.

Types of kits include, but are not limited to, packaged biomarker-specific probe and primer sets (e.g., TaqMan probe/primer sets), arrays/microarrays, biomarker-specific antibodies, biomarker-specific beads, which further contain one or more probes, primers, or other reagents for detecting one or more biomarkers of the present disclosure.

In certain non-limiting embodiments, the present disclosure provides for a kit for determining whether an anti-cancer effect is likely to be produced in a cancer by an EZH2 inhibitor, comprising a means for detecting the presence of a BAP1 nucleic acid biomarker.

In a specific, non-limiting embodiment, a kit may comprise a pair of oligonucleotide primers, suitable for polymerase chain reaction (PCR) or nucleic acid sequencing, for detecting the nucleic acid biomarker(s) to be identified. A pair of primers may comprise nucleotide sequences complementary to a biomarker set forth above, and be of sufficient length to selectively hybridize with said biomarker. Alternatively, the complementary nucleotides may selectively hybridize to a specific region in close enough proximity 5' and/or 3' to the biomarker position to perform PCR and/or sequencing. Multiple biomarker-specific primers may be included in the kit to simultaneously detect more than one biomarker. The kit may also comprise one or more polymerases, reverse transcriptase and nucleotide bases, wherein the nucleotide bases can be further detectably labeled.

In certain non-limiting embodiments, a primer may be at least about 10 nucleotides or at least about 15 nucleotides or at least about 20 nucleotides in length and/or up to about 200 nucleotides or up to about 150 nucleotides or up to about 100 nucleotides or up to about 75 nucleotides or up to about 50 nucleotides in length. Non-limiting examples of primers are provided in Table 1. For example, but not by way of limitation, a primer of the present disclosure can comprise one or more of the sequences disclosed in Table 1.

In a further non-limiting embodiment, the oligonucleotide primers may be immobilized on a solid surface, substrate or support, for example, on a nucleic acid microarray, wherein the position of each oligonucleotide primer bound to the solid surface or support is known and identifiable. The oligonucleotides can be affixed to a substrate, such as glass, plastic, paper, nylon or other type of membrane, filter, chip, bead, or any other suitable solid support. The polynucleotides can be synthesized directly on the substrate, or synthesized separate from the substrate and then affixed to the substrate. The arrays are prepared using known methods.

In a specific, non-limiting embodiment, a kit may comprise at least one nucleic acid probe, suitable for in situ hybridization or fluorescent in situ hybridization, for detecting the biomarker(s) to be identified. Such kits will generally comprise one or more oligonucleotide probes that have specificity for various biomarkers. Means for testing multiple biomarkers may optionally be comprised in a single kit.

In certain embodiments, the kits may comprise containers (including microliter plates suitable for use in an automated implementation of the method), each with one or more of the various reagents (typically in concentrated form) utilized in the methods, including, for example, pre-fabricated microarrays, buffers, the appropriate nucleotide triphosphates (e.g., dATP, dCTP, dGTP and dTTP, or rATP, rCTP, rGTP and UTP), reverse transcriptase, DNA polymerase, RNA polymerase, and one or more probes and primers of the present disclosure (e.g., appropriate length poly(T) or random primers linked to a promoter reactive with the RNA polymerase).

In non-limiting embodiments, the present disclosure provides for a kit for determining whether an anti-cancer effect is likely to be produced in a cancer by an EZH2 inhibitor, comprising a means for detecting the levels of a BAP1 protein biomarker.

In non-limiting embodiments, a kit may comprise at least one antibody, or antigen-binding fragment thereof, for immunodetection of the biomarker(s) to be identified. Antibodies, both polyclonal and monoclonal, specific for a biomarker, may be prepared using conventional immunization techniques, as will be generally known to those of skill in the art. The immunodetection reagents of the kit may include detectable labels that are associated with, or linked to, the given antibody or antigen itself. Such detectable labels include, for example, chemiluminescent or fluorescent molecules (rhodamine, fluorescein, green fluorescent protein, luciferase, Cy3, Cy5, or ROX), radiolabels (³H, ³⁵S, ³²P, ¹⁴C or ¹³¹I) or enzymes (alkaline phosphatase, horseradish peroxidase).

In a further non-limiting embodiment, the one or more biomarker-specific antibodies may be provided bound to a solid support, such as a column matrix, an array or well of a microtiter plate. Alternatively, the support may be provided as a separate element of the kit.

In certain non-limiting embodiments, where the measurement means in the kit employs an array, the set of biomarkers set forth above may constitute at least 10 percent or at least 20 percent or at least 30 percent or at least 40 percent or at least 50 percent or at least 60 percent or at least 70 percent or at least 80 percent of the species of biomarkers represented on the microarray.

In certain non-limiting embodiments, a kit of the present disclosure may contain one or more probes, primers, antibodies or other detection reagents for detecting the expression level of EZH2 in the sample. For example, a kit can contain an antibody, or fragment thereof, for the detection of protein expression level of EZH2 in a biological sample.

In certain non-limiting embodiments, a kit of the present disclosure may contain one or more probes, primers, antibodies or other detection reagents for detecting the expression level of SUZ12 in the sample. For example, a kit can contain an antibody, or fragment thereof, for the detection of protein expression level of SUZ12 in a biological sample.

In certain non-limiting embodiments, a kit of the present disclosure may contain one or more probes, primers, antibodies or other detection reagents for detecting the expression level of L3MBTL2 in the sample. For example, a kit can contain an antibody, or fragment thereof, for the detection of protein expression level of L3MBTL2 in a biological sample.

A kit may further contain means for allowing comparison between the biomarker level within the cancer sample and the biomarker level in a reference control sample. For example, but not by way of limitation, a kit of the present disclosure may contain one or more probes, primers, antibodies or other detection reagents for detecting a reference protein or mRNA, which can be used to normalize the expression levels of the one or more biomarkers from the samples to allow comparison. Non-limiting examples of a reference protein, *e.g.,* a housekeeping protein, include alpha- or beta-tubulin, actin, cofilin, vinculin and GADPH.

In certain non-limiting embodiments, a kit can further include instructions for using the kit to detect the biomarker of interest. For example, the instructions can describe that the absence and/or a lower expression of a BAP1 biomarker, set forth herein, in a cancer sample from a patient, as compared to a reference control level, is indicative of an increased possibility of producing an anti-cancer effect in the cancer by an EZH2 inhibitor.

In certain embodiments, a kit of the present disclosure can further include one or more EZH2 inhibitors. Non-limiting examples of EZH2 inhibitors are disclosed in section 5.2.

The following examples are offered to more fully illustrate the disclosure, but are not to be construed as limiting the scope thereof.

### 6. EXAMPLE 1: LOSS OF BAP1 RESULTS IN INCREASED EZH2 EXPRESSION AND ACTIVITY IN VITRO.

In this example, the mechanism by which loss of BAP1 activity results in disease states was investigated *in vitro.*

### 6.1. RESULTS

SET2 cells were transduced with shRNA targeting BAP1 to reduce BAP1 expression *in vitro.* Reduction in BAP1 expression, as validated by western blot, resulted in an increase in the trimethylation of Histone 3 at K27 (H3K27me3) (FIGURE 1). *See also* Figure 9A. BAP1 protein expression was depleted in BaF3 cells, with confirmation of BAP1 loss by western blot, and histone mass spectrometry was performed. BAP1 knockdown in BaF3 cells revealed an increase in H3K27me3 (FIGURE 1).

BAP1 has been shown to be highly mutated in solid tumors (Carbone et al., 2012) such as in malignant mesothelioma and uveal melanoma. In mesothelioma cells, which had mutations in BAP1, *e.g.,* H28 homozygous deletion, H2452 homozygous missense and H226 deletion mutations, upregulation of EZH2 expression compared to cells with wild-type BAP1 activity was observed (FIGURE 2). Furthermore, *in vitro* overexpression of BAP1 in 293T cells resulted in the reduction of EZH2 and SUZ12 expression, whereas loss of BAP1 expression resulted in the upregulation of SUZ12 expression (FIGURE 3).

### 6.2. DISCUSSION

As described above, the loss of BAP1 protein expression caused an increase in H3K27me3, a repressive chromatin mark placed by EZH2, and an increase in the expression of EZH2 and SUZ12 in *in vitro* cancer cell line systems. EZH2 inhibitors are currently being tested clinically in lymphoma patients with EZH2-activating mutations. Therefore, BAP1 mutation status could assist in identifying patients that may respond to treatment with EZH2 inhibitors, which may be effective in extending survival in BAP1-mutant patients.

### 7. EXAMPLE 2: LOSS OF BAP1 RESULTS IN INCREASED EZH2 EXPRESSION AND ACTIVITY IN VIVO.

### 7.1 METHODS AND MATERIALS

### Primers:

**Table 1**

| **Gene** | | **Genotyping Primers (mouse)** |
|---|---|---|
| *Bap1* up | | ACTGCAGCAATGTGGATCTG (SEQ ID NO: 1) |
| *Bap1* down | | GAAAAGGTCTGACCCAGATCA (SEQ ID NO: 2) |
| *Bap1* flox F | | GCGCAACGCAATTAATGATA (SEQ ID NO: 3) |
| *Bap1* flox R | | CAGTGTCCAGAATGGCTCAA (SEQ ID NO: 4) |
| | | |

| **Gene** | **Mutagenesis Primers (human)** | |
|---|---|---|
| *BAP1 C91A sense* | CCACCAGCTGATACCCAACTCTGCTGCAACTCATGC (SEQ ID NO: 5) | |
| *BAP1 C91A antisense* | GCATGAGTTGCAGCAGAGTTGGGTATCAGCTGGTGG (SEQ ID NO: 6) | |
| | | |

| **Gene** | | **Mouse qPCR Primers** |
|---|---|---|
| *Ezh2* F | | AGCACAAGTCATCCCGTTAAAG (SEQ ID NO: 7) |
| *Ezh2* R | | AATTCTGTTGTAAGGGCGACC (SEQ ID NO: 8) |
| *Suz12* F | | GGCTGACCACGAGCTTTTC (SEQ ID NO: 9) |
| *Suz12* R | | TGGTGCGATAAGATTTCGAGTTC (SEQ ID NO: 10) |
| *Bap1* F | | GTTGGTGGATGACACGTCTG (SEQ ID NO:1 1) |
| *Bap1* R | | CTCAGGACTGAAGCCTTTGG (SEQ ID NO: 12) |
| *Actin B* F | | GATCTGGCACCACACCTTCT (SEQ ID NO: 13) |
| *Actin B* R | | CCATCACAATGCCTGTGGTA (SEQ ID NO: 14) |
| *HoxA5* F | | GCTCAGCCCCAGATCTACC (SEQ ID NO: 15) |
| *HoxA5* R | | GGCATGAGCTATTTCGATCC (SEQ ID NO: 16) |
| *HoxA6* F | | CCCTGTTTACCCCTGGATG (SEQ ID NO: 17) |
| *HoxA6* R | | ACCGACCGGAAGTACACAAG (SEQ ID NO: 18) |
| *HoxA8* F | | CTTCTCCAGTTCCAGCGTCT (SEQ ID NO: 19) |
| *HoxA8* R | | AGGTAGCGGTTGAAATGGAA (SEQ ID NO: 20) |
| *HoxA9* F | | ATGCTTGTGGTTCTCCTCCA (SEQ ID NO: 21) |
| *HoxA9* R | | GTTCCAGCGTCTGGTGTTTT (SEQ ID NO: 22) |
| | | |

| **Gene** | | **Human qPCR Primers** |
|---|---|---|
| *E-CAD* F | | GACCGGTGCAATCTTCAAA (SEQ ID NO: 23) |
| *E-CAD* R | | TTGACGCCGAGAGCTACAC (SEQ ID NO: 24) |
| *HPRT* F | | CATTATGCCGAGGATTTGG (SEQ ID NO: 25) |
| *HPRT* R | | GCAAGTCTTTCAGTCCTGT (SEQ ID NO: 26) |
| *BAP1* F | | CGATCCATTTGAACAGGAAGA (SEQ ID NO: 27) |
| *BAP1* R | | CTCGTGGAAGATTTCGGTGT (SEQ ID NO: 28) |
| | | |

| **Gene** | **ChIP qPCR Primers (human)** | |
|---|---|---|
| *EZH2-1* F | AGCTGACTCAAGCTGCTTGT (SEQ ID NO: 29) | |
| *EZH2-1* R | CAGGAAACCTGAGATTTTCA (SEQ ID NO: 30) | |
| *EZH2-2* F | CTCAGGACAG TTCTGTTTGG (SEQ ID NO: 31) | |
| *EZH2-2* R | TCTGACTTAGTTGGAGAACT (SEQ ID NO: 32) | |
| *SUZ12-1* R | TGAATACAGATGCAGTTATAAGAGAGA (SEQ ID NO: 33) | |
| *MORC3* F | CATCTTCCCCAAGCTCCCAAT (SEQ ID NO: 34) | |
| *MORC3* R | GAGCGAGCTACAAAGCCAGGA (SEQ ID NO: 35) | |
| *E2F6* F | CCTGTTCCCTTCCTCTGGAA (SEQ ID NO: 36) | |
| *E2F6* R | CGACGCAGACGGAAAAAGAG (SEQ ID NO: 37) | |
| *PHF20* F | TGAGTGGGGACTTCGTGTTC (SEQ ID NO: 38) | |
| *PHF20* R | GACCAACCGACAGAAGGACT (SEQ ID NO: 39) | |
| *JAM2* F | TCCACCCCTAGGCTGAAAAG (SEQ ID NO: 40) | |
| *JAM2* R | GATCGGCTTTGTGTCTGGTC (SEQ ID NO: 41) | |

**Animals:** All animals were housed at Memorial Sloan Kettering Cancer Center. All animal procedures were completed in accordance with the Guidelines for the Care and Use of Laboratory Animals and were approved by the Institutional Animal Care and Use Committees at Memorial Sloan Kettering Cancer Center.

**Generation of Bapl-deficient and *Bap1*/*Ezh2-*deficient mice:** Embryonic stem cells targeting exons 6-12 *of Bap1* were obtained from the European Conditional Mouse Consortium. A *Frt*-flanked premature stop cassette containing a lacZ and neomycin cassette was inserted upstream. ES cell clones were expanded and injected into primary blastocysts. Generated mice were crossed to the germline *Flp-*deleter (The Jackson Laboratory) to excise the *Frt*-flanked cassette. These mice were subsequently crossed to the IFN-α-inducible *Mx1-cre* transgenic mice (The Jackson Laboratory) to assess the effects of inducible loss *of Bap1* in the hematopoietic system. *Bap1 fl*/*fl, Bap1 fl*/+ and *Bap1*+/+ littermate mice were genotyped by PCR with the primers BAP1-up (actgcagcaatgtggatctg (SEQ ID NO. 1)), BAP1-down (gaaaaggtctgacccagatca (SEQ ID NO. 2)) using the following parameters: 95°C for 10min, followed by 40 cycles of 94°C for 10s, 65°C for 40s, and 72°C for 1min, and then 72°C for 5min. The WT allele was detected at 300 bp while the floxed allele was detected at 500 bp PCR. Excision after IFN-α-induction was confirmed by a PCR with primers to detect the floxed and excised band: BAP1-F (actgcagcaatgtggatctg (SEQ ID NO. 1)), BAP1-F2 (gcgcaacgcaattaatgata (SEQ ID NO. 3)), and BAP1-R (cagtgtccagaatggctcaa (SEQ ID NO. 4)), using the same PCR parameters listed above. *Mx1-Cre-Bap1 f*/*f* mice were crossed to *Ezh2 f*/*f* mice 12. *Mx-cre Bap1 f*/*f* conditional and *Bap1 f*/*f* control mice received four intraperitoneal injections of polyI:polyC of 200 µL of a 1 mg/mL solution. Two weeks after excision, peripheral blood was collected via retroorbital bleeding using heparinized icrohematocrit capillary tubes (Thermo Fisher Scientific). Excision was confirmed and peripheral blood counts were obtained using a HemaVet according to standard manufacturer's instruction. Formalin-fixed paraffin-embedded tissue sections were stained with hematoxylin and eosin (H&E). Deletion *of Bap1* was confirmed by genomic excision PCR and Western blot analysis. Tails were submitted to the Transnetyx genotyping service (Cordova, TN) for qPCR-based genotyping for floxed and excised *Ezh2* alleles. Excision was confirmed by Western blot.

**Xenografts and *in vivo* EPZ011989 administration:** Groups of 10 week old NOD-SCID mice were injected subcutaneously in the flank with 6-10×10⁶ mesothelioma cell lines (MSTO-211H, Meso10, H226 and H2452) in a 1:1 mixture of matrigel and media. When tumors reached a size of approximately 60-80mm³, treatment with either vehicle (0.5% NaCMC+0.1% Tween-80 in water) or EPZ011989 was initiated. Either EPZ011989 or vehicle were given orally BID at a concentration of 500 mg/kg for the duration of the experiment. Tumor volumes were assessed in three dimensions using a caliper. Tumors or lung tissue were extracted following treatment and utilized for Western blotting to assess target inhibition. Preestablished criteria were generated to exclude mice in xenograft experiments if tumors did not form after implantation (75% smaller than the mean of the implanted animals from the same group. Animals were not excluded from drug trials. For all xenograft drug studies, tumor size was followed for 10 days and mice were randomized at this point for tumor size. The genetic *Bap1* KO EPZ011989 trial was conducted with randomization utilizing CBC analysis 3 weeks after polyI:polyC and confirming that WBC count averages were equivalent in both vehicle and treated groups. Five animals per group were treated orally with either vehicle (described above) or 500 mg/kg EPZ011989 BID for 16 days. Researchers were not blinded in these experiments.

**Histological analyses:** Mice were sacrificed and autopsied, and then dissected tissue samples were fixed for 24 hours in 4% paraformaldehyde, dehydrated, and embedded in paraffin. Paraffin blocks were sectioned at 4 µm and stained with H&E, Ki67, E-Cadherin, or TUNEL. Images were acquired using an Axio Observer A1 microscope (Carl Zeiss).

**Cell culture:** 293T cells were cultured in Dulbecco's modified Eagle's medium (DMEM) supplemented with 10% fetal bovine serum (FBS) and nonessential amino acids. Human leukemia cell lines (SET2) and human mesothelioma cell lines (JMN, Met5a, MSTO-211H, H2373, H226, H2452) were cultured in RPMI-1640 medium supplemented with 10% FBS. MSTO-211H was obtained from ATCC while the remaining mesothelioma lines were generous gifts from Prasad Adusumilli.

**RNA isolation, SMARTer amplification, Proton Transcriptome sequencing and analysis:** Bone marrow cells were FACS sorted for GMPs (Lin⁻c⁻ Kit⁺Sca1⁻CD34⁺Fcγ⁺) using the FACS Aria. Total RNA from 200-500K cells was extracted using TRIzol RNA Isolation Reagents (cat#15596-026, Life Technologies). Quality of RNA was ensured before amplification by analyzing 20-50 pg of each sample using the RNA 6000 pico kit and a bioAnalyzer (Agilent). 10 ng of high quality (RIN>8) total RNA was subsequently amplified using the SMARTER® Universal Low Input RNA Kit for Sequencing (Clonetech Laboratory, cat# 634940) according to instructions provided by the manufacturer. Amplified material underwent whole transcriptome Library preparation according to the Ion Total RNA-Seq Kit v2 protocol (Life Technologies), with 16 cycles of PCR. Samples were barcoded and template-positive ION PI™ ION SPHERE™ Particles (ISPs) were prepared using the ion one touch system II and ION PI™ Template OT2 200kit v2 Kit (Life Technologies). Enriched particles were sequenced on a Proton sequencing system using 200 bp version 2 chemistry. An average of 70 to 80 million reads per sample were generated and 76 to 82% of the reads mapped to mRNA bases. RAW output BAMs were converted back to FASTQ using PICARD Sam2Fastq. Then the reads are first mapped to the mouse genome using rnaStar. The genome used was MM9 with junctions from ENSEMBL (Mus_musculus.NCBIM37.67) and a read overhang of 49. Then any unmapped reads were mapped to MM9 using BWA MEM (version 0.7.5a). The two mapped BAMs were then merged and sorted and gene level counts were computed using htseq-count (options -s y -m intersection-strict) and the same gene models (Mus_musculus.NCBIM37.67). Raw data was uploaded to the GEO database with the following accession number: GSE61360.

**Histone extraction, histone ELISAs, histone Western blots, and histone LC/MS:** Histones were extracted by standard extraction techniques or overnight using the Active Motif Histone Extraction Minikit (40026). Histone ELISAs were conducted using the trimethyl K27 Elisa Kit (Active Motif, 53106) normalized to a H3K27me3 standard curve and total H3 protein. Histone Western blots were conducted with 3-5 µg of histones. For Histone LC/MS, 12 million control and *Bap1* KO cells were lysed, nuclei were isolated and histones were extracted using 0.4N H2SO4 and chemically derivatized using propionic anhydride, as previously described 26. Histones were then digested with trypsin and separated by nano-liquid chromatography (75 µm i.d., 15 cm long, packed with MagicC18aq media, dp 3 µ) coupled to a TSQ Quantum Ultra mass spectrometer. Data were analyzed with Skyline 27 and relative quantification was performed by peak area.

**Chromatin preparation and immunoprecipitation, ChIP Library preparation and sequencing, and analysis of ChIP-Seq data:** Bone marrow cells were enriched for c-Kit+ cells using the EasySep Mouse Hematopoietic cell Enrichment Kit (Stem Cell Technologies, 19756). 5x10⁶ cells were fixed in a 1% methanol-free formaldehyde solution and then resuspended in SDS lysis buffer. Lysates were sonicated in an E220 focused-ultrasonicator (Covaris) to a desired fragment size distribution of 100-500 base pairs. IP reactions were performed using anti-trimethyl H3K27 (Cell Signaling, 9733), antimonomethyl H4K20 (Abcam, 9051), and IgG (Santa Cruz, 2027) each on approximately 400,000 cells as previously described (Krivtsov et al., 2008). ChIP assays were processed on an SX-8G IP-STAR Compact Automated System (Diagenode) using a Direct ChIP protocol as described elsewhere (O'Geen et al., 2011). Eluted chromatin fragments were then decrosslinked and the DNA fragments purified using Agencourt AMPure XP beads (Beckman Coulter).

Barcoded libraries were prepared from the ChIP-enriched and input DNA using a NEBNext ChIP-Seq Library Prep Master Mix Set for Illumina (New England Biolabs) and TruSeq Adaptors (Illumina) according to manufacturer's instructions on an SX-8G IP-STAR Compact Automated System (Diagenode). Phusion High-Fidelity DNA Polymerase (New England Biolabs) and TruSeq PCR Primers (Illumina) were used to amplify the libraries, which were then purified to remove adaptor dimers using AMPure XP beads and multiplexed on the HiSeq 2000 (Illumina).

Reads were quality and adapter-trimmed using 'trim_galore' before aligning to mouse assembly mm9 with bowtie2 using the default parameters. Aligned reads with the same start position and orientation were collapsed to a single read before subsequent analysis. Density profiles were created by extending each read to the average library fragment size and then computing density using the BEDTools suite. Enriched regions were discovered using MACS 1.4 with default parameters, and scored against matched input libraries. All genome browser tracks and read density tables were normalized to sequencing depth. For comparison of ChIP-seq samples in control and KO conditions, the signals of three replicates per condition were tested using either the Mann-Whitney U test or the t-test. Cluster analysis was performed on normalized count data in Matlab with the kmeans clustering package. Motif analysis was performed in Homer using default parameters for the findMotifsGenome program.

**Western blot and immunoprecipitation:** Cells were lysed for Western blot and immunoprecipitation experiments in the following buffer: 150 mM NaCl, 20 mM Tris (pH 7.4), 5 mM EDTA, 1% Triton, protease arrest (EMD) and phosphatase inhibitors (Calbiochem). To perform immunoprecipitations in the presence of benzonase, cells were lysed in the BC-300 buffer: 20 mM Tris (pH 7.4), 10% glycerol, 300 mM KCl, 0.1% NP-40. The cleared lysate was treated with MgCl2 to 2.5 mM and benzonase was added at 1250 U/mL. The lysate was incubated for 1 hour with rotation and the reaction was terminated by adding 5 mM EDTA. DNA digestion was confirmed by running lysate on an ethidium bromide gel before setting up the immunoprecipitation experiment. Antibodies used included: BAP1 (C-4; Santa Cruz sc-28383), EZH2 (Active Motif, 39933, Active Motif, 39901, or Millipore, 07-689), SUZ12 (Abcam, Ab12073), ASXL1 (N-13; Santa Cruz sc-85283), L3MBTL2 (Active Motif, 39569), Myc-Tag (Cell Signaling, 2276), Tubulin (Sigma, T9026), H3K27me3 (Abcam, 6002 or Millipore, 07-449), H3 (Abcam, Ab1791), and H4K20me1 (Abcam, Ab9051).

**Flow cytometry analyses and antibodies:** Surface marker staining of live bone marrow and spleen cells was conducted by first lysing cells with ammonium chloride-potassium bicarbonate lysis buffer and washing cells with phosphate buffered saline (PBS). Cells were stained with antibodies in PBS for 20 minutes on ice. For hematopoietic stem and progenitor staining, cells were stained with a lineage cocktail including CD4 (RM4-5), CD3 (17A2), B220 (RA3-6B2), NK1.1 (PK136), Gr-1 (RB6-8C5), Cd11b (M1/70), and Ter119, allowing for mature lineage exclusion from the analysis. Cells were also stained with antibodies against c-Kit (2B8), Sca-1 (D7), FcγRII/III (2.4G2), and CD34 (RAM34). To assess the composition of the mature mononuclear cells, Mac1, Gr-1, B220, and CD4/CD3 were used. Cell cycle analysis was conducted by staining cells with the hematopoietic stem and progenitor mix described above. Cells were fixed using the FIX and PERM kit (Invitrogen cat# GAS-003). Cells were stained with Ki67 after fixation and then stained with DAPI before analysis on the LSR Fortessa.

**Plasmids:** The cDNA full-length clone of human FLAG-L3MBTL2 was obtained from Addgene (Plasmid 28232). The Myc-His tagged ubiquitin construct was a generous gift from Xuejun Jiang. The cDNA human full-length clone of HA-FLAG BAP1 was obtained from Addgene (Plasmid 22539). The 3X FLAG-tagged BAP1 construct was a generous gift from Marc Ladanyi. Deubiquitinase mutant constructs (C91A, C91S) were generated using Agilent site-directed mutagenesis kits and confirmed by full-length DNA sequencing. Short-hairpin RNAs were obtained from the RNAi Consortium (TRC) in a pLKO. 1 puromycin vector. Sequences for the short-hairpins were as follows: human BAP1 (TRC Oligo IDs: TRCN0000078702 and TRCN0000078698), mouse BAP1 (TRCN0000030719 and TRCN0000030720), human L3MBTL2 (TRCN0000021724 and TRCN0000021726) and a control pLKO.1-puromycin vector encoding an shRNA for luciferase (shLUC).

**Ubiquitin assays:** HEK293T cells were seeded in a 10-cm dish and 24 hours later were transduced with 4 µg of a Myc-His-Ubi expression construct and control, 1 µg L3MBTL2 and/or 1-10 µg BAP1-GFP overexpression constructs. Forty-eight hours after the transfection, cells were lysed in a Guanidine HCl based lysis buffer: 6 M guanidine, 0.1 M NaH2PO4, 10 mM Tris, pH 8.0, and 10 mM BME. His-Ubi proteins were purified by incubation by 20 µL of Ni-NTA agarose (Qiagen) for 4 hours at room temperature. Beads were washed sequentially with 1 mL of 4 wash buffers: buffer A 6 M guanidine, 0.1 M NaH2PO4, 10 mM Tris, pH 8.0, 10 mM BME, and 0.2% Triton-X, buffer B 8 M urea, 0.1 M NaH2PO4, 10 mM Tris, pH 8.0, 10 mM BME, and 0.2% Triton-X, buffer C 0.1 M NaH2PO4, 10 mM Tris, pH 6.3, 10 mM BME, and 0.2% Triton-X, and buffer D 0.1 M NaH2PO4, 10 mM Tris, pH 6.3, 10 mM BME, and 0.1% Triton-X. All buffers were supplemented with 15 mM imidazole. His-tagged proteins were purified from the beads by boiling with 2x SDS Laemmli buffer supplemented with imidazole. Proteins were then analyzed by Western blot.

***In vitro* colony forming assays:** Cells were sorted for Lin⁻c-Kit⁺Sca1⁺ cells using the FACSAria. 100 cells were plated in duplicate in methylcellulose (MethoCult GF M3434, Stem Cell Technologies). Colonies were counted 14 days after plating and colonies were collected by washing with PBS. Cells were then lysed for RNA and histone extraction.

**Transient Transfection:** 293T cells were transfected with indicated constructs with X-treme gene transfection reagent (Roche). Protein and/or histones were extracted 48-72 hours after transfection.

**Invasion Assays:** Mesothelioma cells (MSTO-211H, H2373, H226 and H2452) were seeded in T75 flasks (100,000 cells). 12 hours later the plated cells were treated with GSK126 (0-2 µM) (Chemitek) and then left to proliferate for 7 days. 250,000 treated cells were then placed on the top of a Matrigel invasion chamber (BD Biosciences, cat no. 354480) in serum free media while the lower chamber contained media with serum. 22 hours later the cells on the bottom of the membrane were stained with crystal violet and quantitated with ImageJ.

**Luciferase Assays:** 293T cells were transiently transfected with the pGL3 EZH2 promoter reporter construct (generous gift from Naomi Goldfinger) and a Switchgear Renilla control construct in addition to EV, BAP1, and L3MBTL2 constructs. Cells were assessed for luciferase activity using the DualLuciferase Reporter Assay System (Promega). Cells were seeded in 24 well plates and were cotransfected with 200 ng pGL3-EZH2-Luciferase, 200 ng of the Renilla luciferase control construct, and 500 ng of experimental constructs. Cells were incubated 48 hours after the transfection, lysed for 15 minutes at room temperature and luciferase activity was assessed on a luminometer. The Firefly luciferase readings were normalized to the Renilla transfection control.

**Statistical Analyses:** The Student t-test with Welch's correction was used to analyze statistical significance unless described in the text. Prizm GraphPad Software was used for statistical calculations. Error was calculated using SEM, *p<0.05, **p<0.005.

### 7.2 RESULTS

Genomic studies identified somatic mutations in the tumor suppressors *ASXL1* and *BAP1* in different malignancies. The Drosophila ASXL1 homolog Asx and the BAP1 homolog Calypso form a complex which removes H2AK119Ub (Scheuermann et al., 2010). However, the BAP1-ASXL1 complex has not been shown to have a role in *BAP1*-mutant transformation. Inactivating mutations in *ASXL1* are most common in myeloid malignancies (Abdel-Wahab et al., 2011; Bejar et al., 2011; Gelsi-Boyer et al., 2009), whereas recurrent *BAP1* mutations are commonly observed in mesothelioma (Bott et al., 2011), renal cell carcinoma (Pena-Llopis et al., 2012), and metastatic uveal melanoma (Harbour et al., 2010) suggesting *BAP1* and *ASXL1* have distinct roles in tumor suppression. These mutational profiles cannot be explained by differential tissue-specific *BAP1* and *ASXL1* expression (FIGURE 5A-C). This Example identifies the mechanisms by which BAP1 loss leads to transformation, independent of ASXL1, and identifies the therapeutic vulnerabilities in *BAP1*-mutant cancer cells.

Recent studies have shown that somatic loss of *Bap1* can promote hematopoietic transformation (Dey et al., 2012). The impact of conditional *Bap1* deletion on gene expression and chromatin state in hematopoietic cells was investigated (FIGURE 5A, B). Conditional deletion *of Bap1* was generated using the scheme shown in FIGURE 5D. MX-Cre, a recombinase that drives *Bap1* deletion in hematopoietic tissues following induction in the adult animal was used. *Bap1* loss led to a fully penetrant myeloproliferative disease with splenomegaly (FIGURE 6A), leukocytosis (FIGURE 5E,F), anemia (FIGURE 5G,H) and granulocyte macrophage progenitors (GMPs) expansion (FIGURE 5I-K). For example, in *Bap1* knockout (KO) mice, the size, *e.g*., weight and length, of the spleen was observed to be larger in size than BAP1 wild-type mice (FIGURE 6A, 10C and 11C). An increase in proliferation and cell cycle progression of *Bap1*-deficient myeloid progenitors was also observed (FIGURE 5L). RNA sequencing analysis revealed the majority of differentially expressed genes in Bapl-deficient GMPs had reduced expression (p-adj<0.001) (FIGURE 7A). Although significant overlap between the set of differentially expressed genes in *Bap1* and *Asxll* KO progenitors was observed, in many cases a paradoxical inverse effect on gene expression was observed (FIGURE 6B). Gene set enrichment analysis (GSEA) identified inversely impacted gene sets enriched in *Bap1* and *Asxl1* KO progenitors (Abdel-Wahab et al., 2013) (FIGURE 7B). *ASXL1* silencing leads to increased expression of *HoxA* cluster genes consistent with reduced PRC2 activity (Abdel-Wahab et al., 2012). By contrast, reduced expression of *HoxA* gene members (FIGURE 6C) and decreased expression of *HoxA* gene signatures in Bapl-deficient cells (FIGURE 7C) were observed. These data demonstrate that loss of *Asxll* and *Bap1* have opposite effects on gene regulation.

ASXL1 directly interacts with the PRC2 complex and *ASXL1* depletion reduces global and site-specific H3K27me3 (Abdel-Wahab et al., 2012). Given the divergent effects of *Asxll* and *Bap1* loss on gene expression, the impact of *Bap1* deletion on H3K27me3 was investigated. H3K27me3 levels were increased in *Bap1* KO cells by histone mass spectrometry (FIGURE 6D), Western blot (FIGURE 6E), and ELISA (FIGURE 8A). H3K27me3 chromatin immunoprecipitation sequencing (ChIP-Seq) revealed a global increase in *Bap1* KO mice (FIGURE 6F), with an increased number of H3K27me3 broad domains (Beguelin et al., 2013) (FIGURE 6G), and increased H3K27me3 broad domain "spreading" into nearby loci (FIGURE 6H). This H3K27me3 increase and spreading is well illustrated within the *HoxA* locus in *Bap1* KO cells (FIGURE 6I). The sites marked with H3K27me3 in *Bap1* KO cells, primarily occurred in gene promoter regions (FIGURE 8B) and genes with H3K27me3-occupied promoters were enriched for enhanced repression (FDR<0.001) (FIGURE 6J). Similar findings in purified GMPs were observed (FIGURE 6K). Genes dysregulated by *Bap1* KO-associated H3K27me3 and gene repression were implicated in EZH2-dependent regulation, lineage commitment/differentiation and proliferation (FIGURE 6L, FIGURE 8C). BAP1 silencing increased H3K27me3 (FIGURE 9A), and re-expression of BAP1 in Bapl-deficient cells reduced H3K27me3 levels (FIGURE 9B). By contrast, a deubiquitinase-deficient BAP1 allele did not reduce H3K27me3 (FIGURE 9C), demonstrating alterations in H3K27me3 are due to BAP1 catalytic activity.

Next, the role of PRC2-mediated H3K27me3 on BAP1-dependent transformation was assessed by investigating the impact of *Ezh2* loss (Su et al., 2003) on transformation *in vivo. Ezh2* deletion reduced H3K27me3 levels in *Bap1*/*Ezh2-*deficient mice compared to *Bap1*-knockout mice (FIGURE 11A). *Ezh2* deletion abrogated the myeloid malignancy induced by *Bap1* loss (FIGURE 10A), with reduced splenomegaly (FIGURE 11B,C), leukocytosis (FIGURE 11D) and anemia (FIGURE 11E). Concomitant *Bap1*/*Ezh2* loss reduced myeloid progenitor expansion (FIGURE 11F), reduced the proportion of Mac1⁺Gr1⁺ myeloid cells (FIGURE 11G) and restored erythroid differentiation (CD71⁺Ter119⁺) (FIGURE 10B). Decreased proliferation of *Bap1*/*Ezh2* deficient progenitors was observed (FIGURE 11H). *Ezh2* haploinsufficiency reduced, but did not abrogate, *Bap1*-deficient myeloproliferation (FIGURE 10C, 10D) consistent with a dose-dependent requirement for *Ezh2.* Consistent with the genetic data, treatment of *Bap1* KO mice with the small molecule inhibitor EPZ011989 (Campbell et al., 2015) decreased H3K27me3, splenomegaly, and white blood cell counts (FIGURE 11I-K). These data demonstrate that PRC2, and specifically *Ezh2* activity, is required for *Bap1*-deficient myeloid transformation.

Next, the mechanism by which *Bap1* deletion increased H3K27me3 levels was investigated. In contrast to the reported interactions between ASXL1 and BAP1 and between ASXL1 and PRC2, an interaction between BAP1 and EZH2 by co-IP was not identified (FIGURE 13A). An increase in mRNA and protein expression of Ezh2 and Suz12 was observed (FIGURE 13B,C) consistent with a role for Bap1 in regulating PRC2 expression. In addition, analysis of sorted Lin⁻Sca⁺Kit⁺ cells (a population containing the hematopoietic stem cells) from the whole bone marrow of *Bap1* KO mice showed significant increases in *Suz12* and *Ezh2* RNA expression compared to wild-type BAP1 mice (FIGURE 17). Further, whole bone marrow western blots revealed an increase in the protein expression of EZH2 and SUZ12 in *Bap1* KO cells compared to control cells (FIGURE 17). Re-expression of BAP1 in *Bap1* KO bone marrow cells reduced *Ezh2* mRNA expression to normal levels (FIGURE 12A). It was hypothesized that *Bap1* loss might directly alter other histone marks, which would then alter chromatin state at key target loci, including EZH2. Histone mass spectrometry revealed a marked decrease in H4K20me1 in *Bap1* KO cells (FIGURE 13D) compared to other measured histone marks (FIGURE 12B). Expression of BAP1, but not ASXL1 or BMI1, increased H4K20me1 at the *EZH2* locus (FIGURE 12C). It was therefore hypothesized that loss of the H4K20me1 mark may have an important role in BAP1-dependent gene expression. SETD8 is the only known methyltransferase that places H4K20me1 (Nishioka et al., 2002). Expression of SETD8 in *BAP1*-mutant mesothelioma cells (H226, H2452) increased apoptosis and reduced proliferation, whereas wild-type (MSTO-211H and Meso10) cells were unaffected (FIGURE 13E,F). SETD8 overexpression in mesothelioma cells decreased EZH2 mRNA and protein expression (FIGURE 13G,H). *BAP1* wild-type cell lines were more sensitive to a SETD8 inhibitor (Blum et al., 2014) (BVT594) than *BAP1-*mutant cell lines (FIGURE 13I).

It was hypothesized that BAP1 deubiquitinates a chromatin modulator that regulates H4K20me1. Analysis of ChIP-Seq data (Abdel-Wahab et al., 2013; Dey et al., 2012) identified a cluster of genes with Bap1 occupancy, but not Asxl1 binding (Cluster 1) and were enriched for an E-box motif (FIGURE 14A,B). Previous studies have shown the atypical polycomb proteins L3MBTL1 and L3MBTL2 bind E-box motifs, and can bind and maintain H4K20me1 (Guo et al., 2009; Qin et al., 2012; Trojer et al., 2011; Trojer et al., 2007). *L3mbtl1*-deficient mice have no overt phenotype (Qin et al., 2010), whereas *L3mbtl2-deficient* mice are embryonic lethal similar in timing to *Bap1* loss (Dey et al., 2012; Qin et al., 2012). Therefore, whether *Bap1* loss led to alterations in L3mbt12 expression was investigated. L3mbt12 protein but not RNA expression was reduced in *Bap1* KO hematopoietic cells (FIGURE 15A,B) and in *BAP1*-mutant mesothelioma cells compared to *BAP1* wild-type mesothelioma cells (FIGURE 13J). L3MBTL2 ubiquitination was reduced in cells expressing BAP1 (FIGURE 13K) and proteasome inhibitor treatment increased L3MBTL2 stability in *BAP1*-mutant cells (FIGURE 15C). L3MBTL2 expression decreased EZH2 protein levels with and without BAP1 co-expression (FIGURE 15D) and expression of BAP1 or L3MBTL2 overexpression reduced EZH2 promoter activity (FIGURE 15E). Conversely, L3MBTL2 silencing increased expression of EZH2 (FIGURE 15F). An enrichment for L3MBTL2 and BAP1 at the *EZH2* locus was observed in cells expressing L3MBTL2 and BAP1 (FIGURE 15G,H). Without being bound to a particular theory, these data suggest that BAP1 and L3MBTL2 interact (FIGURE 15I) and co-occupy the *EZH2* locus. *BAP1* loss leads to reduced L3MBTL2 stability and increased *EZH2* transcriptional output (FIGURE 13L).

Analysis of TCGA data revealed that EZH2 mRNA expression was increased in mesothelioma (FIGURE 16A). Next, whether EZH2 inhibition might inhibit the survival of *BAP1*-mutant mesothelioma cell lines was assessed. EZH2 silencing induced apoptosis in *BAP1*-mutant cell lines, whereas wild-type cell lines continued to proliferate (FIGURE 16B,C). EZH2 silencing abrogated *in vivo* tumor formation of *BAP1*-mutant but not wild-type cell lines (FIGURE 16D). Overexpression of EZH2 in *BAP1* wild-type cell lines increased proliferation (FIGURE 20A) and sensitivity to EZH2 inhibition (FIGURE 20B). *BAP1*-mutant cell lines were more sensitive to EZH2 inhibition (EPZ011989) *in vitro* both in 2D (FIGURE 16E) and 3D culture (FIGURE 16F). Next, the impact of EZH2 inhibition *in vivo* was assessed. EZH2 inhibition significantly reduced *BAP1*-mutant tumor size compared to vehicle treated mice (FIGURE 16G), whereas wild-type tumors were less/not responsive to EZH2 inhibition (FIGURE 16H), despite similar effects on H3K27me3. EZH2 inhibition abrogated pulmonary metastasis in a *BAP1*-mutant mesothelioma cell line with metastatic potential (FIGURE 16G) consistent with a role for BAP1/EZH2 in metastasis (Harbour et al., 2010). EZH2 inhibition reduced invasion and increased E-Cadherin expression *in vitro* (FIGURE 20C-D). Next, the impact of EZH2 inhibition using the BAP1 inhibitor GSK126 was assessed. *BAP1-*mutant mesothelioma cells were injected into the flank of NOD-SCID mice, and then initiated treatment with either vehicle or 150 mg/kg GSK126 after tumor formation. EZH2 inhibition significantly reduced tumor size compared to vehicle treated mice (FIGURE 21A and FIGURE 4). GSK126 treatment significantly attenuated H3K27me3 in *BAP1*-mutant cells *in vivo* (FIGURE 21B). Pathologic analysis revealed that EZH2 inhibition was associated with reduced Ki67 staining and increased TUNEL staining (FIGURE 21C). These data indicate that EZH2 represents a potential therapeutic target in *BAP1*-mutant cancer cells.

The identification of oncogenic *EZH2* mutations (Morin et al., 2010; Morin et al., 2011; Pasqualucci et al., 2011) has led to the development of mutant-specific epigenetic therapies. However, most mutations in epigenetic regulators result in loss-of-function, such that they do not represent tractable direct therapeutic targets. EZH2 inhibitors have recently entered clinical trials (McCabe et al., 2012) and the disclosed data suggest that *BAP1* loss results in a mutation-specific dependency in PRC2 that should be further explored in preclinical and clinical studies. These data resonate with recent studies suggesting a role for PRC2 inhibition in *SWI*/*SNF-*mutant rhabdoid tumors (Alimova et al., 2013; Knutson et al., 2013) and analyses showing *BAP1* mutations are mutually exclusive with SWI/SNF mutations (Wilson et al., 2010). These data suggest that detailed studies of mutations in epigenetic regulators can be used to inform the development of therapies that reverse mutant-specific effects on epigenetic state in different malignant contexts.

### 7.3 DISCUSSION

BAP1 and ASXL1 interact to form a polycomb deubiquitinase complex that can remove monoubiquitin from histone H2A lysine 119 (H2AK119Ub). However, *BAP1* and *ASXL1* are mutated in distinct cancer types, consistent with independent roles in regulating epigenetic state and in malignant transformation. In this Example, it is demonstrated that *Bap1* loss results in increased trimethylated histone H3 lysine 27 (H3K27me3), elevated *Ezh2* expression, and enhanced repression of Polycomb Repressive Complex 2 (PRC2) targets. These findings are in contrast to the reduction in H3K27me3 seen with *Asxl1* loss. Conditional deletion of *Bap1* and *Ezh2 in vivo* abrogates the myeloid progenitor expansion induced by *Bap1* loss alone. Loss of Bap1 results in a marked decrease in H4K20 monomethylation (H4K20me1). Consistent with a role for H4K20me1 in EZH2 transcriptional regulation, expression of SETD8, the H4K20me1 methyltransferase, reduces EZH2 expression and abrogates the proliferation *of BAP1-*mutant cells. Further, mesothelioma cells that lack BAP1 are sensitive to EZH2 pharmacologic inhibition, suggesting a novel therapeutic approach for *BAP1*-mutant malignancies.

### 8. REFERENCES

Abdel-Wahab, O., Adli, M., LaFave, L.M., Gao, J., Hricik, T., Shih, A.H., Pandey, S., Patel, J.P., Chung, Y.R., Koche, R., et al. (2012). ASXL1 mutations promote myeloid transformation through loss of PRC2-mediated gene repression. Cancer Cell 22, 180-193.
Abdel-Wahab, O., Gao, J., Adli, M., Dey, A., Trimarchi, T., Chung, Y.R., Kuscu, C., Hricik, T., Ndiaye-Lobry, D., Lafave, L.M., et al. (2013). Deletion of Asxl1 results in myelodysplasia and severe developmental defects in vivo. J Exp Med 210, 2641-2659.
Abdel-Wahab, O., Pardanani, A., Patel, J., Wadleigh, M., Lasho, T., Heguy, A., Beran, M., Gilliland, D.G., Levine, R.L., and Tefferi, A. (2011). Concomitant analysis of EZH2 and ASXL1 mutations in myelofibrosis, chronic myelomonocytic leukemia and blast-phase myeloproliferative neoplasms. Leukemia 25, 1200-1202.
Alimova, I., Birks, D.K., Harris, P.S., Knipstein, J.A., Venkataraman, S., Marquez, V.E., Foreman, N.K., and Vibhakar, R. (2013). Inhibition of EZH2 suppresses self-renewal and induces radiation sensitivity in atypical rhabdoid teratoid tumor cells. Neuro Oncol 15, 149-160.
Beguelin, W., Popovic, R., Teater, M., Jiang, Y., Bunting, K.L., Rosen, M., Shen, H., Yang, S.N., Wang, L., Ezponda, T., et al. (2013). EZH2 is required for germinal center formation and somatic EZH2 mutations promote lymphoid transformation. Cancer Cell 23, 677-692.
Bejar, R., Stevenson, K., Abdel-Wahab, O., Galili, N., Nilsson, B., Garcia-Manero, G., Kantarjian, H., Raza, A., Levine, R.L., Neuberg, D., et al. (2011). Clinical effect of point mutations in myelodysplastic syndromes. The New England journal of medicine 364, 2496-2506.
Blum, G., Ibanez, G., Rao, X., Shum, D., Radu, C., Djaballah, H., Rice, J.C., and Luo, M. (2014). Small-molecule inhibitors of SETD8 with cellular activity. ACS Chem Biol 9, 2471-2478.
Bott, M., Brevet, M., Taylor, B.S., Shimizu, S., Ito, T., Wang, L., Creaney, J., Lake, R.A., Zakowski, M.F., Reva, B., et al. (2011). The nuclear deubiquitinase BAP1 is commonly inactivated by somatic mutations and 3p21.1 losses in malignant pleural mesothelioma. Nature genetics 43, 668-672.
Campbell, J.E., Kuntz, K.W., Knutson, S.K., Warholic, N.M., Keilhack, H., Wigle, T.J., Raimondi, A., Klaus, C.R., Rioux, N., Yokoi, A., et al. (2015). EPZ011989, A Potent, Orally-Available EZH2 Inhibitor with Robust in
Vivo Activity. ACS Med Chem Lett 6, 491-495.
Dey, A., Seshasayee, D., Noubade, R., French, D.M., Liu, J., Chaurushiya, M.S., Kirkpatrick, D.S., Pham, V.C., Lill, J.R., Bakalarski, C.E., et al. (2012). Loss of the tumor suppressor BAP1 causes myeloid transformation. Science 337, 1541-1546.
Garcia, B.A., Mollah, S., Ueberheide, B.M., Busby, S.A., Muratore, T.L., Shabanowitz, J., and Hunt, D.F. (2007). Chemical derivatization of histones for facilitated analysis by mass spectrometry. Nat Protoc 2, 933-938.
Gelsi-Boyer, V., Trouplin, V., Adelaide, J., Bonansea, J., Cervera, N., Carbuccia, N., Lagarde, A., Prebet, T., Nezri, M., Sainty, D., et al. (2009). Mutations of polycomb-associated gene ASXL1 in myelodysplastic syndromes and chronic myelomonocytic leukaemia. British journal of haematology 145, 788-800.
Guo, Y., Nady, N., Qi, C., Allali-Hassani, A., Zhu, H., Pan, P., Adams-Cioaba, M.A., Amaya, M.F., Dong, A., Vedadi, M., et al. (2009). Methylation-state-specific recognition of histones by the MBT repeat protein L3MBTL2. Nucleic Acids Res 37, 2204-2210.
Harbour, J.W., Onken, M.D., Roberson, E.D., Duan, S., Cao, L., Worley, L.A., Council, M.L., Matatall, K.A., Helms, C., and Bowcock, A.M. (2010). Frequent mutation of BAP1 in metastasizing uveal melanomas. Science 330, 1410-1413.
Knutson, S.K., Warholic, N.M., Wigle, T.J., Klaus, C.R., Allain, C.J., Raimondi, A., Porter Scott, M., Chesworth, R., Moyer, M.P., Copeland, R.A., et al. (2013). Durable tumor regression in genetically altered malignant rhabdoid tumors by inhibition of methyltransferase EZH2. Proc Natl Acad Sci USA 110, 7922-7927.
Krivtsov, A.V., Feng, Z., Lemieux, M.E., Faber, J., Vempati, S., Sinha, A.U., Xia, X., Jesneck, J., Bracken, A.P., Silverman, L.B., et al. (2008). H3K79 methylation profiles define murine and human MLL-AF4 leukemias. Cancer Cell 14, 355-368.
Lara-Astiaso, D., et al. Immunogenetics. Chromatin state dynamics during blood formation. Science 345, 943-949 (2014).
Love, M.I., Huber, W., and Anders, S. (2014). Moderated estimation of fold change and dispersion for RNA-seq data with DESeq2. Genome biology 15, 550.
MacLean, B., Tomazela, D.M., Shulman, N., Chambers, M., Finney, G.L., Frewen, B., Kern, R., Tabb, D.L., Liebler, D.C., and MacCoss, M.J. (2010). Skyline: an open source document editor for creating and analyzing targeted proteomics experiments. Bioinformatics 26, 966-968.
McCabe, M.T., Ott, H.M., Ganji, G., Korenchuk, S., Thompson, C., Van Aller, G.S., Liu, Y., Graves, A.P., Della Pietra, A., 3rd, Diaz, E., et al. (2012). EZH2 inhibition as a therapeutic strategy for lymphoma with EZH2-activating mutations. Nature 492, 108-112.
Morin, R.D., Johnson, N.A., Severson, T.M., Mungall, A.J., An, J., Goya, R., Paul, J.E., Boyle, M., Woolcock, B.W., Kuchenbauer, F., et al. (2010). Somatic mutations altering EZH2 (Tyr641) in follicular and diffuse large B-cell lymphomas of germinal-center origin. Nature genetics 42, 181-185.
Morin, R.D., Mendez-Lago, M., Mungall, A.J., Goya, R., Mungall, K.L., Corbett, R.D., Johnson, N.A., Severson, T.M., Chiu, R., Field, M., et al. (2011). Frequent mutation of histone-modifying genes in non-Hodgkin lymphoma. Nature 476, 298-303.
Nishioka, K., Rice, J.C., Sarma, K., Erdjument-Bromage, H., Werner, J., Wang, Y., Chuikov, S., Valenzuela, P., Tempst, P., Steward, R., et al. (2002). PR-Set7 is a nucleosome-specific methyltransferase that modifies lysine 20 of histone H4 and is associated with silent chromatin. Mol Cell 9, 1201-1213.
O'Geen, H., Echipare, L., and Farnham, P.J. (2011). Using ChIP-seq technology to generate high-resolution profiles of histone modifications. Methods Mol Biol 791, 265-286.
Pasqualucci, L., Trifonov, V., Fabbri, G., Ma, J., Rossi, D., Chiarenza, A., Wells, V.A., Grunn, A., Messina, M., Elliot, O., et al. (2011). Analysis of the coding genome of diffuse large B-cell lymphoma. Nat Genet 43, 830-837.
Pena-Llopis, S., Vega-Rubin-de-Celis, S., Liao, A., Leng, N., Pavia-Jimenez, A., Wang, S., Yamasaki, T., Zhrebker, L., Sivanand, S., Spence, P., et al. (2012). BAP1 loss defines a new class of renal cell carcinoma. Nat Genet 44, 151-759.
Phung, Y.T., Barbone, D., Broaddus, V.C., and Ho, M. (2011). Rapid generation of in vitro multicellular spheroids for the study of monoclonal antibody therapy. Journal of Cancer 2, 507-514.
Qin, J., Van Buren, D., Huang, H.S., Zhong, L., Mostoslavsky, R., Akbarian, S., and Hock, H. (2010). Chromatin protein L3MBTL1 is dispensable for development and tumor suppression in mice. J Biol Chem 285, 27767-27775.
Qin, J., Whyte, W.A., Anderssen, E., Apostolou, E., Chen, H.H., Akbarian, S., Bronson, R.T., Hochedlinger, K., Ramaswamy, S., Young, R.A., et al. (2012). The polycomb group protein L3mbt12 assembles an atypical PRC1-family complex that is essential in pluripotent stem cells and early development. Cell Stem Cell 11, 319-332.
Scheuermann, J.C., de Ayala Alonso, A.G., Oktaba, K., Ly-Hartig, N., McGinty, R.K., Fraterman, S., Wilm, M., Muir, T.W., and Muller, J. (2010). Histone H2A deubiquitinase activity of the Polycomb repressive complex PR-DUB. Nature 465, 243-247.
Skarnes, W.C., Rosen, B., West, A.P., Koutsourakis, M., Bushell, W., Iyer, V., Mujica, A.O., Thomas, M., Harrow, J., Cox, T., et al. (2011). A conditional knockout resource for the genome-wide study of mouse gene function. Nature 474, 337-342.
Su, I.H., Basavaraj, A., Krutchinsky, A.N., Hobert, O., Ullrich, A., Chait, B.T., and Tarakhovsky, A. (2003). Ezh2 controls B cell development through histone H3 methylation and Igh rearrangement. Nat Immunol 4, 124-131.
Suraokar, M.B., Nunez, M.I., Diao, L., Chow, C.W., Kim, D., Behrens, C., Lin, H., Lee, S., Raso, G., Moran, C., et al. (2014). Expression profiling stratifies mesothelioma tumors and signifies deregulation of spindle checkpoint pathway and microtubule network with therapeutic implications. Annals of oncology : official journal of the European Society for Medical Oncology / ESMO 25, 1184-1192.
Trojer, P., Cao, A.R., Gao, Z., Li, Y., Zhang, J., Xu, X., Li, G., Losson, R., Erdjument-Bromage, H., Tempst, P., et al. (2011). L3MBTL2 protein acts in concert with PcG protein-mediated monoubiquitination of H2A to establish a repressive chromatin structure. Mol Cell 42, 438-450.
Trojer, P., Li, G., Sims, R.J., 3rd, Vaquero, A., Kalakonda, N., Boccuni, P., Lee, D., Erdjument-Bromage, H., Tempst, P., Nimer, S.D., et al. (2007). L3MBTL1, a histone-methylation-dependent chromatin lock. Cell 129, 915-928.
Wilson, B.G., Wang, X., Shen, X., McKenna, E.S., Lemieux, M.E., Cho, Y.J., Koellhoffer, E.C., Pomeroy, S.L., Orkin, S.H., and Roberts, C.W. (2010). Epigenetic antagonism between polycomb and SWI/SNF complexes during oncogenic transformation. Cancer Cell 18, 316-328.

## Claims

1. An EZH2 inhibitor for use in the treatment of a cancer in a subject in need thereof, wherein the expression of a BAP1 biomarker in the cancer of the subject is absent or expressed at a lower level than a BAP1 reference control level,
wherein the BAP1 reference control level is the expression level of the BAP1 biomarker in a reference control sample;
wherein the cancer is selected from the group consisting of malignant mesotheliomas, uveal melanomas, renal cell carcinoma, cutaneous melanomas, lung cancer, breast cancer, ovarian cancer, non-melanoma skin cancer, meningioma, cholangiocarcinoma, leiomyosarcoma, neuroendocrine tumors, pancreatic cancer, paraganglioma, malignant fibrous histiocytoma, melanocytic BAP1-mutated atypical intradermal tumors (MBAITs), acute myeloid leukemia, myelodysplastic syndromes, and bladder cancer; and
wherein the EZH2 inhibitor is selected from the group consisting of UNC1999, 3-deazaneplanocin A, EI1, EPZ-6438, EPZ-5676, GSK343, EPZ005687, EPZ011989, and GSK126.

2. An EZH2 inhibitor for use in a method for treating a cancer in a subject in need thereof, comprising determining the expression of a BAP1 biomarker in one or more cells of the cancer of the subject, where if the BAP1 biomarker is absent or expressed at lower levels in the cancer, as compared to a BAP1 reference control level, then administering a therapeutically effective amount of the EZH2 inhibitor to the subject to produce an anti-cancer effect,
wherein the BAP1 reference control level is the expression level of the BAP1 biomarker in a reference control sample;
wherein the cancer is selected from the group consisting of malignant mesotheliomas, uveal melanomas, renal cell carcinoma, cutaneous melanomas, lung cancer, breast cancer, ovarian cancer, non-melanoma skin cancer, meningioma, cholangiocarcinoma, leiomyosarcoma, neuroendocrine tumors, pancreatic cancer, paraganglioma, malignant fibrous histiocytoma, melanocytic BAP1-mutated atypical intradermal tumors (MBAITs), acute myeloid leukemia, myelodysplastic syndromes, and bladder cancer; and
wherein the EZH2 inhibitor is selected from the group consisting of UNC1999, 3-deazaneplanocin A, EI1, EPZ-6438, EPZ-5676, GSK343, EPZ005687, EPZ011989, and GSK126.

3. The EZH2 inhibitor for use of claim 2, further comprising determining, in the one or more cells of the cancer of the subject, the expression level of EZH2 and/or SUZ12, where if the BAP1 biomarker is absent or expressed at a lower level than a BAP1 reference control level, and if the expression of EZH2 is increased compared to an EZH2 reference control level and/or the expression of SUZ12 is increased compared to a SUZ12 reference control level, then administering to the subject the EZH2 inhibitor.

4. The EZH2 inhibitor for use of any one of claims 1-3, wherein the cancer is a malignant mesothelioma, a uveal melanoma, or a renal cell carcinoma.

5. The EZH2 inhibitor for use of any one of claims 1-3, wherein the expression of the BAP1 biomarker is determined by immunofluorescence, Western Blot, in situ hybridization, polymerase chain reaction, or using a reagent which specifically binds with the BAP1 biomarker, for example wherein the reagent is an antibody or an antigen binding fragment thereof.

6. The EZH2 inhibitor for use of any one of claims 1-5, wherein the EZH2 inhibitor is EPZ-6438.

7. A method for determining whether an anti-cancer effect is likely to be produced in a cancer by an EZH2 inhibitor, comprising determining in a sample of a cancer the expression level of an BAP1 biomarker, where if the BAP1 biomarker is absent or expressed at a lower level in the cancer, as compared to a BAP1 reference control level, it is more likely that the EZH2 inhibitor would have an anti-cancer effect on the cancer,
wherein the BAP1 reference control level is the expression level of the BAP1 biomarker in a reference control sample;
wherein the cancer is selected from the group consisting of malignant mesotheliomas, uveal melanomas, renal cell carcinoma, cutaneous melanomas, lung cancer, breast cancer, ovarian cancer, non-melanoma skin cancer, meningioma, cholangiocarcinoma, leiomyosarcoma, neuroendocrine tumors, pancreatic cancer, paraganglioma, malignant fibrous histiocytoma, melanocytic BAP1-mutated atypical intradermal tumors (MBAITs), acute myeloid leukemia, myelodysplastic syndromes, and bladder cancer; and
wherein the EZH2 inhibitor is selected from the group consisting of UNC1999, 3-deazaneplanocin A, EI1, EPZ-6438, EPZ-5676, GSK343, EPZ005687, EPZ011989, and GSK126.

8. The method of claim 7, wherein the BAP1 biomarker is a BAP1 protein biomarker or a BAP1 nucleic acid biomarker.

9. The method of claim 7 or 8, wherein the expression of the BAP1 biomarker is determined by immunofluorescence or Western Blot.

10. A method of predicting the sensitivity of a cancer to an EZH2 inhibitor, comprising determining in a sample of a cancer the expression level of a BAP1 protein biomarker in cells of the sample, wherein if the BAP1 biomarker is absent or reduced in expression level compared to a BAP1 reference control level, then the cancer is predicted to be sensitive to an EZH2 inhibitor,
wherein the BAP1 reference control level is the expression level of the BAP1 biomarker in a reference control sample;
wherein the cancer is selected from the group consisting of malignant mesotheliomas, uveal melanomas, renal cell carcinoma, cutaneous melanomas, lung cancer, breast cancer, ovarian cancer, non-melanoma skin cancer, meningioma, cholangiocarcinoma, leiomyosarcoma, neuroendocrine tumors, pancreatic cancer, paraganglioma, malignant fibrous histiocytoma, melanocytic BAP1-mutated atypical intradermal tumors (MBAITs), acute myeloid leukemia, myelodysplastic syndromes, and bladder cancer; and
wherein the EZH2 inhibitor is selected from the group consisting of UNC1999, 3-deazaneplanocin A, EI1, EPZ-6438, EPZ-5676, GSK343, EPZ005687, EPZ011989, and GSK126.

11. The method of any one of claims 7-11, wherein the EZH2 inhibitor is EPZ-6438.

12. Use of a kit in the method of any one of claims 7-9, wherein the kit comprises a means for detecting the BAP1 biomarker.

13. The use of claim 12, wherein the means for detecting the BAP1 biomarker comprises one or more packaged primers, probe, arrays/microarray, biomarker-specific antibody and/or bead, optionally wherein the means for detecting the BAP1 biomarker comprises one or more antibodies, or antigen binding fragment thereof, for detecting the BAP1 biomarker.

14. The use of claim 12 or 13, wherein the kit further comprises one or more primers, probe, arrays/microarray, biomarker-specific antibody and/or bead for detecting EZH2 expression, L3MBTL2 expression and/or SUZ12 expression.

## Patentansprüche

1. EZH2-Inhibitor zur Verwendung beim Behandeln eines Krebses bei einem Subjekt, das dies benötigt, wobei die Expression eines BAP1-Biomarkers bei dem Krebs des Subjekts fehlt oder auf einem niedrigeren Pegel exprimiert wird als einem BAP1-Referenzkontrollpegel,
wobei der BAP1-Referenzkontrollpegel der Expressionspegel des BAP1-Biomarkers in einer Referenzkontrollprobe ist;
wobei der Krebs ausgewählt ist aus der Gruppe bestehend aus malignen Mesotheliomen, Uvealmelanomen, Nierenzellkarzinomen, Hautmelanomen, Lungenkrebs, Brustkrebs, Eierstockkrebs, Nicht-Melanom-Hautkrebs, Meningiom, Cholangiokarzinom, Leiomyosarkom, neuroendokrinen Tumoren, Bauchspeicheldrüsenkrebs, Paragangliom, malignem fibrösem Histiozytom, melanozytären BAP1-mutierten atypischen intradermalen Tumoren (MBAITs), akuter myeloischer Leukämie, myelodysplastischen Syndromen und Blasenkrebs; und
wobei der EZH2-Inhibitor ausgewählt ist aus der Gruppe bestehend aus UNC1999, 3-Deazanplanocin A, EI1, EPZ-6438, EPZ-5676, GSK343, EPZ005687, EPZ011989 und GSK126.

2. EZH2 Hemmstoff zur Verwendung in einem Verfahren zum Behandeln eines Krebses bei einem Subjekt, das dies benötigt, umfassend das Bestimmen der Expression eines BAP1-Biomarkers in einer oder mehreren Krebszellen des Subjekts, wobei, wenn der BAP1-Biomarker bei dem Krebs fehlt oder im Vergleich zu einem BAP1-Referenzkontrollpegel auf einem niedrigeren Pegel exprimiert wird, dann eine therapeutisch wirksame Menge des EZH2-Inhibitors an das Subjekt verabreicht wird, um einen Antikrebseffekt herbeizuführen,
wobei der BAP1-Referenzkontrollpegel der Expressionspegel des BAP1-Biomarkers in einer Referenzkontrollprobe ist;
wobei der Krebs ausgewählt ist aus der Gruppe bestehend aus malignen Mesotheliomen, Uvealmelanomen, Nierenzellkarzinomen, Hautmelanomen, Lungenkrebs, Brustkrebs, Eierstockkrebs, Nicht-Melanom-Hautkrebs, Meningiom, Cholangiokarzinom, Leiomyosarkom, neuroendokrinen Tumoren, Bauchspeicheldrüsenkrebs, Paragangliom, malignem fibrösem Histiozytom, melanozytären BAP1-mutierten atypischen intradermalen Tumoren (MBAITs), akuter myeloischer Leukämie, myelodysplastischen Syndromen und Blasenkrebs; und
wobei der EZH2-Inhibitor ausgewählt ist aus der Gruppe bestehend aus UNC1999, 3-Deazanplanocin A, EI1, EPZ-6438, EPZ-5676, GSK343, EPZ005687, EPZ011989 und GSK126.

3. EZH2-Inhibitor zur Verwendung nach Anspruch 2, ferner umfassend das Bestimmen, in der einen oder den mehreren Krebszellen des Subjekts, des Expressionspegels von EZH2 und/oder SUZ12, wobei, wenn der BAP1-Biomarker fehlt oder im Vergleich zu einem BAP1-Referenzkontrollpegel auf einem niedrigeren Pegel exprimiert wird, und wenn die Expression von EZH2 im Vergleich zu einem EZH2-Referenzkontrollpegel erhöht ist und/oder wenn die Expression von SUZ12 im Vergleich zu einem SUZ12-Referenzkontrollpegel erhöht ist, dem Subjekt dann der EZH2-Inhibitor verabreicht wird.

4. EZH2- Inhibitor zur Verwendung nach einem der Ansprüche 1 bis 3, wobei der Krebs ein malignes Mesotheliom, ein Uvealmelanom oder ein Nierenzellkarzinom ist.

5. EZH2- Inhibitor zur Verwendung nach einem der Ansprüche 1 bis 3, wobei die Expression des BAP1-Biomarkers bestimmt wird durch Immunfluoreszenz, Western Blot, In-situ-Hybridisierung, Polymerasekettenreaktion oder unter Verwendung eines Reagenzes, das spezifisch an den BAP1-Biomarker bindet, zum Beispiel wobei das Reagenz ein Antikörper oder ein antigenbindendes Fragment davon ist.

6. EZH2- Inhibitor zur Verwendung nach einem der Ansprüche 1-5, wobei der EZH2-Inhibitor EPZ-6438 ist.

7. Verfahren zum Bestimmen, ob eine krebsbekämpfende Wirkung bei einem Krebs durch einen EZH2-Inhibitor wahrscheinlich hervorgerufen wird, umfassend das Bestimmen, in einer Probe eines Krebses, des Expressionspegels eines BAP1-Biomarkers, wobei, wenn der BAP1-Biomarker bei dem Krebs fehlt oder auf einem niedrigeren Pegel exprimiert wird als einem BAP1-Referenzkontrollpegel, es wahrscheinlicher ist, dass der EZH2-Inhibitor eine krebsbekämpfende Wirkung auf den Krebs hat,
wobei der BAP1-Referenzkontrollpegel der Expressionspegel des BAP1-Biomarkers in einer Referenzkontrollprobe ist;
wobei der Krebs ausgewählt ist aus der Gruppe bestehend aus malignen Mesotheliomen, Uvealmelanomen, Nierenzellkarzinomen, Hautmelanomen, Lungenkrebs, Brustkrebs, Eierstockkrebs, Nicht-Melanom-Hautkrebs, Meningiom, Cholangiokarzinom, Leiomyosarkom, neuroendokrinen Tumoren, Bauchspeicheldrüsenkrebs, Paragangliom, malignem fibrösem Histiozytom, melanozytären BAP1-mutierten atypischen intradermalen Tumoren (MBAITs), akuter myeloischer Leukämie, myelodysplastischen Syndromen und Blasenkrebs; und
wobei der EZH2-Inhibitor ausgewählt ist aus der Gruppe bestehend aus UNC1999, 3-Deazanplanocin A, EI1, EPZ-6438, EPZ-5676, GSK343, EPZ005687, EPZ011989 und GSK126.

8. Verfahren nach Anspruch 7, wobei der BAP1-Biomarker ein BAP1-Proteinbiomarker oder ein BAP1-Nukleinsäurebiomarker ist.

9. Verfahren nach Anspruch 7 oder 8, wobei die Expression des BAP1-Biomarkers durch Immunofluoreszenz oder Western-Blot bestimmt.

10. Verfahren zum Vorhersagen der Empfindlichkeit eines Krebses auf einen EZH2-Inhibitor, umfassend das Bestimmen, in einer Probe von einem Krebs, des Expressionspegels eines BAP1-Proteinbiomarkers in Zellen der Probe, wobei, wenn der BAP1-Biomarker fehlt oder auf einem niedrigeren Pegel exprimiert wird als einem BAP1-Referenzkontrollpegel, vorhergesagt wird, dass der Krebs empfindlich auf einen EZH2-Inhibitor reagiert,
wobei der BAP1-Referenzkontrollpegel der Expressionspegel des BAP1-Biomarkers in einer Referenzkontrollprobe ist;
wobei der Krebs ausgewählt ist aus der Gruppe bestehend aus malignen Mesotheliomen, Uvealmelanomen, Nierenzellkarzinomen, Hautmelanomen, Lungenkrebs, Brustkrebs, Eierstockkrebs, Nicht-Melanom-Hautkrebs, Meningiom, Cholangiokarzinom, Leiomyosarkom, neuroendokrinen Tumoren, Bauchspeicheldrüsenkrebs, Paragangliom, malignem fibrösem Histiozytom, melanozytären BAP1-mutierten atypischen intradermalen Tumoren (MBAITs), akuter myeloischer Leukämie, myelodysplastischen Syndromen und Blasenkrebs; und
wobei der EZH2-Inhibitor ausgewählt ist aus der Gruppe bestehend aus UNC1999, 3-Deazanplanocin A, EI1, EPZ-6438, EPZ-5676, GSK343, EPZ005687, EPZ011989 und GSK126.

11. Verfahren nach einem der Ansprüche 7-11, wobei der EZH2-Inhibitor EPZ-6438 ist.

12. Verwendung eines Kits in dem Verfahren nach einem der Ansprüche 7-9, wobei das Kit ein Mittel zum Nachweisen des BAP1-Biomarkers umfasst.

13. Verwendung nach Anspruch 12, wobei das Mittel zum Erfassen des BAP1-Biomarkers einen oder mehrere verpackte Primer, Sonden, Arrays/Mikroarrays, Biomarkerspezifische Antikörper und/oder Beads umfasst, optional wobei das Mittel zum Erfassen des BAP1-Biomarkers einen oder mehrere Antikörper oder antigenbindende Fragmente davon zum Nachweisen des BAP1-Biomarkers umfasst.

14. Verwendung nach Anspruch 12 oder 13, wobei das Kit ferner einen oder mehrere Primer, Sonden, Arrays/Mikroarrays, Biomarkers-spezifische Antikörper und/oder Beads zum Nachweisen von EZH2, zum Nachweisen von L3MBTL2 und/oder zum Nachweisen von SUZ12 umfasst.

## Revendications

1. Inhibiteur de l'EZH2 pour une utilisation dans le traitement d'un cancer chez un sujet qui en a besoin, dans lequel l'expression d'un biomarqueur BAP1 dans le cancer du sujet est absente ou exprimée à un niveau inférieur à un niveau de contrôle de référence du BAP1,
dans lequel le niveau de contrôle de référence du BAP1 est le niveau d'expression du biomarqueur BAP1 dans un échantillon contrôle de référence ;
dans lequel le cancer est choisi dans le groupe consistant en les mésothéliomes malins, les mélanomes uvéaux, le carcinome des cellules rénales, les mélanomes cutanés, un cancer du poumon, un cancer du sein, un cancer des ovaires, un cancer de la peau non-mélanome, un méningiome, un cholangiocarcinome, un léiomyosarcome, des tumeurs neuroendocrines, un cancer du pancréas, un paragangliome, un histiocytome fibreux malin, une tumeur mélanocytaire intradermique atypique causée par une mutation de BAP1 (MBAIT), une leucémie myéloïde aiguë, des syndromes myélodysplasiques et un cancer de la vessie ; et
dans lequel l'inhibiteur de l'EZH2 est choisi dans le groupe consistant en UNC1999, la 3-déazanéplanocine A, EI1, EPZ-6438, EPZ-5676, GSK343, EPZ005687, EPZ0111989 et GSK126.

2. Inhibiteur de l'EZH2 pour une utilisation dans un procédé de traitement d'un cancer chez un sujet qui en a besoin, comprenant la détermination de l'expression d'un biomarqueur BAP1 dans une ou plusieurs cellules du cancer du sujet, où, si le biomarqueur BAP1 est absent ou exprimé à des niveaux inférieurs dans le cancer, par comparaison avec un niveau de contrôle de référence du BAP1, puis l'administration d'une quantité thérapeutiquement efficace de l'inhibiteur de l'EZH2 au sujet pour produire un effet anti-cancéreux,
dans lequel le niveau de contrôle de référence du BAP1 est le niveau d'expression du biomarqueur BAP1 dans un échantillon contrôle de référence ;
dans lequel le cancer est choisi dans le groupe consistant en les mésothéliomes malins, les mélanomes uvéaux, le carcinome des cellules rénales, les mélanomes cutanés, un cancer du poumon, un cancer du sein, un cancer des ovaires, un cancer de la peau non-mélanome, un méningiome, un cholangiocarcinome, un léiomyosarcome, des tumeurs neuroendocrines, un cancer du pancréas, un paragangliome, un histiocytome fibreux malin, une tumeur mélanocytaire intradermique atypique causée par une mutation de BAP1 (MBAIT), une leucémie myéloïde aiguë, des syndromes myélodysplasiques et un cancer de la vessie ; et
dans lequel l'inhibiteur de l'EZH2 est choisi dans le groupe consistant en UNC1999, la 3-déazanéplanocine A, EI1, EPZ-6438, EPZ-5676, GSK343, EPZ005687, EPZ0111989 et GSK126.

3. Inhibiteur de l'EZH2 pour une utilisation selon la revendication 2, comprenant en outre la détermination, dans une ou plusieurs cellules du cancer du sujet, du niveau d'expression de l'EZH2 et/ou du SUZ12, où, si le biomarqueur BAP1 est absent ou exprimé à un niveau inférieur à un niveau de contrôle de référence du BAP1, et si l'expression de l'EZH2 est accrue par comparaison avec un niveau de contrôle de référence de l'EZH2 et/ou que l'expression du SUZ12 soit accrue par comparaison avec un niveau de contrôle de référence du SUZ12, puis l'administration au sujet de l'inhibiteur de l'EZH2.

4. Inhibiteur de l'EZH2 pour une utilisation selon l'une quelconque des revendications 1 à 3, dans lequel le cancer est un mésothéliome malin, un mélanome uvéal ou un carcinome des cellules rénales.

5. Inhibiteur de l'EZH2 pour une utilisation selon l'une quelconque des revendications 1 à 3, dans lequel l'expression du biomarqueur BAP1 est déterminée par immunofluorescence, transfert de Western, hybridation in situ, réaction en chaîne par polymérase, ou par utilisation d'un réactif qui se lie spécifiquement au biomarqueur BAP1, par exemple dans lequel le réactif est un anticorps ou un fragment de liaison à l'antigène de celui-ci.

6. Inhibiteur de l'EZH2 pour une utilisation selon l'une quelconque des revendications 1 à 5, dans lequel l'inhibiteur de l'EZH2 est l'EPZ-6438.

7. Procédé de détermination si un effet anticancéreux est susceptible d'être produit dans un cancer par un inhibiteur de l'EZH2, comprenant la détermination dans un échantillon d'un cancer du niveau d'expression d'un biomarqueur BAP1, où si le biomarqueur BAP1 est absent ou exprimé à un niveau inférieur dans le cancer, par comparaison avec un niveau de contrôle de référence du BAP1, il est plus vraisemblable que l'inhibiteur de l'EZH2 aurait un effet anticancéreux sur le cancer,
dans lequel le niveau de contrôle de référence du BAP1 est le niveau d'expression du biomarqueur BAP1 dans un échantillon contrôle de référence ;
dans lequel le cancer est choisi dans le groupe consistant en les mésothéliomes malins, les mélanomes uvéaux, le carcinome des cellules rénales, les mélanomes cutanés, un cancer du poumon, un cancer du sein, un cancer des ovaires, un cancer de la peau non-mélanome, un méningiome, un cholangiocarcinome, un léiomyosarcome, des tumeurs neuroendocrines, un cancer du pancréas, un paragangliome, un histiocytome fibreux malin, une tumeur mélanocytaire intradermique atypique causée par une mutation de BAP1 (MBAIT), une leucémie myéloïde aiguë, des syndromes myélodysplasiques et un cancer de la vessie ; et
dans lequel l'inhibiteur de l'EZH2 est choisi dans le groupe consistant en UNC1999, la 3-déazanéplanocine A, EI1, EPZ-6438, EPZ-5676, GSK343, EPZ005687, EPZ0111989 et GSK126.

8. Procédé selon la revendication 7, dans lequel le biomarqueur BAP1 est un biomarqueur protéine de BAP1 ou un biomarqueur acide nucléique de BAP1.

9. Procédé selon la revendication 7 ou 8, dans lequel l'expression du biomarqueur BAP1 est déterminée par immunofluorescence ou transfert de Western.

10. Procédé de prédiction de la sensibilité d'un cancer à un inhibiteur de l'EZH2, comprenant la détermination dans un échantillon d'un cancer du niveau d'expression d'un biomarqueur protéine de BAP1 dans des cellules de l'échantillon, dans lequel si le biomarqueur BAP1 est absent ou réduit pour ce qui est du niveau d'expression par comparaison avec un niveau de contrôle de référence du BAP1, alors le cancer est prédit être sensible à un inhibiteur de l'EZH2,
dans lequel le niveau de contrôle de référence du BAP1 est le niveau d'expression du biomarqueur BAP1 dans un échantillon contrôle de référence ;
dans lequel le cancer est choisi dans le groupe consistant en les mésothéliomes malins, les mélanomes uvéaux, le carcinome des cellules rénales, les mélanomes cutanés, un cancer du poumon, un cancer du sein, un cancer des ovaires, un cancer de la peau non-mélanome, un méningiome, un cholangiocarcinome, un léiomyosarcome, des tumeurs neuroendocrines, un cancer du pancréas, un paragangliome, un histiocytome fibreux malin, une tumeur mélanocytaire intradermique atypique causée par une mutation de BAP1 (MBAIT), une leucémie myéloïde aiguë, des syndromes myélodysplasiques et un cancer de la vessie ; et
dans lequel l'inhibiteur de l'EZH2 est choisi dans le groupe consistant en UNC1999, la 3-déazanéplanocine A, EI1, EPZ-6438, EPZ-5676, GSK343, EPZ005687, EPZ0111989 et GSK126.

11. Procédé selon l'une quelconque des revendications 7-11, dans lequel l'inhibiteur de l'EZH2 est l'EPZ-6438.

12. Utilisation d'un kit dans le procédé selon l'une quelconque des revendications 7-9, dans laquelle le kit comprend un moyen pour détecter le biomarqueur BAP1.

13. Utilisation selon la revendication 12, dans laquelle le moyen de détection du biomarqueur BAP1 comprend un ou plusieurs amorces empaquetées, sondes, réseaux/micro-réseaux, anticorps spécifiques de biomarqueur et/ou perles, éventuellement dans laquelle le moyen de détection du biomarqueur BAP1 comprend un ou plusieurs anticorps ou fragments de liaison à l'antigène de ceux-ci, pour la détection du biomarqueur BAP1.

14. Utilisation selon la revendication 12 ou 13, dans laquelle le kit comprend en outre un ou plusieurs amorces, sondes, réseaux/micro-réseaux, anticorps spécifiques de biomarqueur et/ou perles pour la détection de l'expression de l'EZH2, de l'expression du L3MBTL2 et/ou de l'expression du SUZ12.
